# FASCICULE DE BREVET EUROPEEN

(11) **EP 1 598 053 B1**
(45) Date de publication et mention de la délivrance du brevet: **20.12.2006**
(21) Numéro de dépôt: 05290943.9
(22) Date de dépôt: 29.04.2005
(51) Int. Cl.: A61K 8/55, A61Q 5/08

(54) **Utilisation pour le décapage de la couleur artificielle des fibres kératiniques d'une composition comprenant au moins une phosphine à titre d'argent réducteur majoritaire**
Utilisation pour le décapage de la couleur artificielle des fibres kératiniques d'une composition comprenant au moins une phosphine à titre d'agent réducteur majoritaire
Use of a composition containing at least one phosphine as majority reducing agent to remove the artificial colour of keratinic fibres

(30) Priorité: 17.05.2004 FR 0405353
(43) Date de publication de la demande: 23.11.2005
(73) Titulaire: L'ORÉAL, 75008 Paris (FR)
(72) Inventeur: Kravtchenko, Sylvain, 92600 Asnières (FR); Livoreil, Aude, 75006 Paris (FR)
(74) Mandataire: Fevrier, Murielle Françoise E.

(56) Documents cités:
- EP-A- 0 943 316
- WO-A-97/11672
- US-A- 3 892 845

## Description

La présente invention a pour objet l'utilisation pour le décapage de la couleur artificielle des fibres kératiniques, et en particulier des fibres kératiniques humaines telles que les cheveux, d'une composition comprenant au moins une phosphine ou l'un de ses sels d'addition avec un acide à titre d'agent réducteur majoritaire.

Il est connu de teindre les fibres kératiniques, et en particulier les fibres kératiniques humaines telles que les cheveux, avec des compositions tinctoriales contenant des colorants d'oxydation et / ou des colorants directs.

La teinture effectuée avec des colorants d'oxydation ou "coloration d'oxydation" est une coloration permanente ; elle comprend à titre de colorants d'oxydation, les précurseurs de coloration d'oxydation et les coupleurs.

Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.

La variété des molécules mises en jeu au niveau des bases d'oxydation et des coupleurs, permet l'obtention d'une riche palette de couleurs.

La teinture effectuée avec des colorants directs donne une coloration semi-permanente ou temporaire ; les colorants directs apportent à la coloration naturelle des cheveux, une modification de couleur plus ou moins marquée résistant éventuellement à plusieurs shampooings.

Les colorants directs classiquement utilisés sont choisis notamment parmi les colorants directs nitrés benzéniques, les colorants directs azoïques, les colorants directs quinoniques et en particulier anthraquinoniques, les colorants directs aziniques, les colorants directs triarylméthaniques, les colorants directs indoaminiques et les colorants directs naturels.

Pour varier les nuances obtenues en coloration d'oxydation, ou les enrichir de reflets, on peut utiliser les colorants d'oxydation en association avec des colorants directs.

Pour des raisons diverses, telles que le souhait de moduler partiellement ou totalement la nuance ainsi conférée à la chevelure par une teinture d'oxydation, une teinture directe, ou le souhait d'éliminer cette coloration, on peut être amené à vouloir détruire partiellement ou totalement les colorants ainsi formés ou amenés dans ou sur le cheveu. On procède alors au décapage de la couleur artificielle des fibres kératiniques.

Ce décapage est en général effectué via des procédés mettant en oeuvre des systèmes oxydants ou réducteurs.

Dans les systèmes oxydants, les agents oxydants classiquement utilisés sont le peroxyde d'hydrogène ou des composés susceptibles de produire des composés peroxygénés par hydrolyse.

Parmi les systèmes réducteurs, on connaît, de part le brevet allemand DE 1 151 242, l'usage de l'acide hydroxyméthanesulfinique à un pH compris entre 7 et 9, pour décolorer des cheveux colorés. On connaît également l'usage du sulfite de sodium (Na₂SO₃) dans les brevets US 2 149 319 et US 3 838 966 et la demande JP-04356413A.

Il est également connu de décaper les fibres kératiniques à l'aide d'agents réducteurs à pH acide. Ainsi, il est connu d'utiliser l'hydroxyméthanesulfinate de sodium comme réducteur des cheveux teints, que l'on mélange au moment de l'emploi avec une solution aqueuse acide. Dans la demande de brevet EP 0 943 316, il est décrit l'utilisation d'une association à pH acide comprenant de l'acide ascorbique et de l'acide alpha-oxocarboxylique pour décaper les cheveux.

Un procédé pour décaper la couleur artificielle des fibres kératiniques est également décrit dans le brevet US 3 892 845 et consiste à appliquer sur les fibres une composition aqueuse comprenant la combinaison de deux types de réducteurs, un réducteur du colorant et un réducteur des liaisons covalentes disulfure de la kératine ; le réducteur du colorant est un hydroxyméthanesulfinate ou un hydrosulfite de zinc, potassium, sodium ou de calcium, et le réducteur de la kératine est notamment l'acide thioglycolique, un bisulfate ou un bisulfite de potassium ou de sodium, le bisulfure de potassium, la thiourée, ou certains composés phosphorés, tels que les phosphines.

Cependant, toutes ces techniques de l'art antérieur n'engendrent pas un décapage des fibres kératiniques suffisamment performant, en particulier pour des nuances fondamentales et les nuances à reflets dorés et cendrés.

Le but de la présente invention est de foumir des compositions pour le décapage de la couleur artificielle des fibres kératiniques qui ne présentent pas les inconvénients des produits de décapage connus de l'art antérieur, en particulier des compositions pour le décapage de la couleur artificielle des fibres kératiniques plus efficaces.

Ce but est atteint avec la présente invention qui a pour objet l'utilisation pour le décapage de la couleur artificielle des fibres kératiniques d'une composition comprenant, dans un milieu cosmétiquement acceptable, au moins une phosphine ou l'un de ses sels d'addition avec un acide à titre d'agent réducteur majoritaire.

La présente invention a également pour objet un procédé de décapage de la couleur artificielle des fibres kératiniques mettant en oeuvre une composition comprenant au moins une phosphine ou l'un de ses sels d'addition avec un acide à titre d'agent réducteur majoritaire.

La présente invention a aussi pour objet un dispositif à plusieurs compartiments permettant de teindre les fibres kératiniques puis de décaper la couleur artificielle des fibres kératiniques.

La présente invention permet d'obtenir le décapage de la couleur artificielle des fibres kératiniques teintes avec une palette large de colorants d'oxydation et / ou de colorants directs, qui n'induisent pas d'éclaircissement de la base naturelle des fibres kératiniques, qui soient plus pratique d'utilisation, par exemple qui ne nécessitent pas de mélange au moment de l'emploi et qui limitent la sensibilisation des fibres kératiniques.

Dans le cadre de la présente invention, on entend par « agent réducteur majoritaire » le fait que dans le cas où la composition contient des agents réducteurs additionnels différents de la phosphine, ceux-ci sont toujours chacuns présents en quantité pondérale inférieure à la quantité pondérale des phosphines.

Dans les définitions ci-dessous, les radicaux hydrocarbonés sont linéaires ou ramifiés, cycliques ou acycliques, saturés ou insaturés, et comprennent, sauf indication contraire, de 1 à 30 atomes de carbone, de préférence de 1 à 10 atomes de carbone.

On entend par radical hydrocarboné insaturé, un radical hydrocarboné comprenant de 2 à 30 atomes de carbone, de préférence de 2 à 10 atomes de carbone, et comprenant une ou plusieurs doubles et /ou triples liaisons.

A titre d'exemples de phosphines utiles dans le cadre de la présente invention, on peut citer la tri(hydroxyméthyl)phosphine ; la tri(hydroxypropyl)phosphine ; la bis(hydroxyméthyl)(phényl)phosphine ; l'allyldiphénylphosphine ; la benzyloxy(diisopropylamino)méthylphosphine ; le 2,2'-bis(dicyclohexylphosphino)-1,1'-binaphtyle ; le 2,2'-bis[bis(3,5-diméthylphénylphosphino)]-1,1'-binaphtyle ; le 1,4-bis[bis(3,5-diméthylphényl)phosphino]butane ; le 1,2-bis[bis(3,5-diméthylphényl)phosphino]éthane ; le bis[bis(3,5-diméthylphényl)phosphino]méthane ; le 1,5-bis[bis(3,5-diméthylphényl)phosphino]pentane ; le 1,3-bis[bis(3,5-diméthylphényl)phosphino]propane ; le 2,2'-bis[bis(3,5-ditrifluorométhylphényl)phosphino]-1,1'-binaphtyle ; le 1,4-bis[bis(3,5-ditrifluorométhylphényl)phosphino]butane ; le 1,2-bis[bis(3,5-ditrifluorométhylphényl)phosphino]éthane ; le bis[bis(3,5-ditrifluorométhylphényl)phosphino]méthane ; le 1,5-bis[bis(3,5-ditrifluorométhylphényl)phosphino]pentane ; le 1,3-bis[bis(3,5-ditrifluorométhylphényl)phosphino]propane ; la bis(3,5-di-tert-butylphényl)chlorophosphine ; la bis(3,5-di-tert-butylphényl)phosphine ; le 1,2-bis(di-tert-butylphosphino)benzène ; le 1,4-bis(di-tert-butylphosphino)butane ; le 1,2-bis(di-tert-butylphosphino)éthane ; le 1,1'-bis(di-tert-butylphosphino)ferrocène ; le 1,3-bis(di-tert-butylphosphinométhyl)benzène ; le 1,3-bis(di-tert-butylphosphino)propane ; le 1,2-bis(dichlorophosphino)benzène ; le 1,3-bis(dichlorophosphino)benzène ; le 1,4-bis(dichlorophosphino)benzène ; le 1,4-bis(dichlorophosphino)butane ; la 1,2-bis(dichlorophosphino)-1,2-diméthylhydrazine ; le 1,2-bis(dichlorophosphino)éthane ; le 1,1'-bis(dichlorophosphino)ferrocène ; le bis(dichlorophosphino)méthane ; le 1,3-bis(dichlorophosphino)propane ; le 1,2-bis(dicyclohexylphosphino)benzène ; le 2,2'-bis(dicyclohexylphosphino)-1,1'-binaphtyle ; le 1,4-bis(dicyclohexylphosphino)butane ; le (2R,3R)-bis(dicyclohexylphosphino)butane ; le (2S,3S)-bis(dicyclohexylphosphino)butane ; le 1,2-bis(dicyclohexylphosphino)éthane ; le bis(dicyclohexylphosphino)méthane ; le 1,3-bis(dicyclohexylphosphino)propane ; la bis(diéthylamino)chlorophosphine ; le bis[2-(4-diéthylaminométhylphényl)-phénylphosphino]éthyl éther ; le 1,2-bis(diéthylphosphino)éthane ; la bis(diisopropylamino)chlorophosphine ; le 1,1'-bis(diisopropylphosphino)ferrocène ; la bis(3,5-diméthyl-4-méthoxyphényl)chlorophosphine ; la bis(3,5-diméthyl-4-méthoxyphényl)phosphine ; la bis(3,5-diméthylphényl)chlorophosphine ; la bis(3,5-diméthylphényl)diéthylaminophosphine ; la bis(3,5-diméthylphényl)phosphine ; le 1,2-bis(diméthylphosphino)benzène ; le 1,4-bis(diméthylphosphino)butane ; le 1,2-bis(diméthylphosphino)éthane ; le bis(diméthylphosphino)méthane ; le 1,3-bis(diméthylphosphino)propane ; le 1,2-bis(diphénylphosphino)benzène ; le 1,3-bis(diphénylphosphino)benzène ; le 1,4-bis(diphénylphosphino)benzène ; le 2,2'-bis(diphénylphosphino)-1,1'-binaphtyle ; le 1,4-bis(diphénylphosphino)butane ; le 1,2-bis(diphénylphosphino)éthane ; le cis-1,2-bis(diphénylphosphino)éthylène ; le trans-1,2-bis(diphénylphosphino)éthylène ; le bis(2-diphénylphosphino)éthyl éther ; la bis(2-diphénylphosphinoéthyl)phénylphosphine ; le 1,1'-bis(diphénylphosphino)ferrocène ; le dichlorure de 1,1'-bis(diphénylphosphino)ferrocènenickel(II) ; le 1,6-bis(diphénylphosphino)hexane ; le bis(diphénylphosphino)méthane ; le 1,5-bis(diphénylphosphino)pentane ; le 1,3-bis(diphénylphosphino)propane ; la bis(3,5-ditrifluorométhyl)chlorophosphine ; la bis(3,5-ditrifluorométhylphényl)phosphine ; le 1,2-bis(ditrifluorométhylphosphino)éthane ; la bis(2-furyl)chlorophosphine ; la bis(2-furyl)phosphine ; la bis(hydroxyméthyl)phénylphosphine ; la bis(4-méthoxyphényl)phénylphosphine ; le 1,2-bis[(2-méthoxyphényl)phénylphosphino]éthane ; la bis(4-méthylphényl)chlorophosphine ; la bis(4-méthylphényl)phosphine ; le 1,2-bis(phénylphosphino)éthane ; le 1,3-bis(phénylphosphino)propane ; le bis-2-[(phényl)(3-pyridyl)phosphinoéthyl] éther ; le 1,2-bis(phosphino)benzène ; le 1,2-bis(phosphino)éthane ; le 1,1'-bis(phosphino)ferrocène ; le bis(phosphino)méthane ; la bis(pyrrolidino)méthylphosphine ; la bis(4-trifluorométhylphényl)phosphine ; le 1,2-bis(trifluorométhyl)phosphino)éthane ; le dichlorure de bis(triméthylphosphine)cobalt ; le dichlorure de bis(triméthylphosphine)cuivre ; le dichlorure de bis(triméthylphosphine)fer ; le dichlorure de bis(triméthylphosphine)zinc ; la tert-butylbis(triméthylsilyl)phosphine ; la butyldichlorophosphine ; la tert-butyldichlorophosphine ; la tert-butyldiéthylphosphine ; la butyldiphénylphosphine ; la tert-butyldiphénylphosphine ; la chlorodüsopropylphosphine ; la 2-chloroéthyldiphénylphosphine ; la cyclohexyldichlorophosphine ; la cyclohexyl(diéthylamino)chlorophosphine ; la cyclohexyl(diméthylamino)chlorophosphine ; la cyclohexyldiphénylphosphine ; la diallylphénylphosphine ; la dibenzylphosphine ; la di-tert-butylchlorophosphine ; les complexes de di-tert-butylchlorophosphine borane ; la dibutylphénylphosphine ; la dibutylphosphine ; la di-tert-butylphénylphosphine ; la di-tert-butylphosphine ; les complexes de di-tert-butylphosphine borane ; le 2-(di-tert-butylphosphino)biphényle ; la dicyclohexylchlorophosphine ; la dicyclohexylphosphine ; les complexes de dicyclohexylphosphine borane ; le 2-(dicyclohexylphosphino)biphényle ; le 2-dicyclohexylphosphino-2'-(N,N-diméthylamino)biphényle ; la diéthylaminodiéthylphosphine ; la diéthylchlorophosphine ; la diéthylphénylphosphine ; la diéthylphosphine ; la diisobutylphosphine; la diméthylaminodichlorophosphine ; la (4-diméthylaminophényl)diphénylphosphine ; la diméthylchlorophosphine ; la diméthylphénylphosphine ; la diméthyl(triméthylsilyl)phosphine ; la diphénylphosphine ; l'acide diphénylphosphinique ; la N-[(diphénylphosphinyl)méthyl]-N-méthylaniline ; la diphénylchlorophosphine ; la diphénylphosphine ; l'acide o-diphénylphosphinobenzoïque ; la diphénylpropylphosphine ; la diphényl(p-tolyl)phosphine ; la diphényl(triméthylsilyl)phosphine ; la diphénylvinylphosphine ; la divinylphénylphosphine ; l'éthyldichlorophosphine ; l'éthyldiphénylphosphine ; l'isopropyldichlorophosphine ; le 2-méthoxy(dichtorophosphino)benzène ; la méthoxydiéthoxyphosphine ; la 4-méthoxyphényl(diéthylamino)chlorophosphine ; la 4-méthoxyphényl(diméthylamino)chlorophosphine ; la (2-méthoxyphényl)méthylphénylphosphine ; le 2-méthoxyphosphinobenzène ; le méthyl-2,3-bis-O-diphénylphosphino-4,6-O-benzylidèneglucopyranoside ; la méthyldichlorophosphine ; la méthyldiphénylphosphine ; la (5-méthyl-2-isopropylcyclohexyl)diphénylphosphine ; la méthylphénylchlorophosphine ; la phénylphosphine ; le 9H-9-phosphabicyclo[3,3,1]nonane (phobane) ; le 9H-9-phosphabicyclo[4,2,1]nonane (phobane) ; la propyldichlorophosphine ; la tétraphénylbiphosphine ; la tri-tert-butylphosphine ; la tricyclohexylphosphine ; la tricyclopentylphosphine ; la triéthylphosphine ; la triisobutylphosphine ; la triisopropylphosphine ; la triméthylphophine ; la tri-n-octylphosphine ; le dibromure de triphénylphosphine ; le dichlorure de triphénylphosphine ; l'hydrobromure de triphénylphosphine ; la tripropylphosphine ; la tris[3,5-bis(trifluorométhyl)phényl]phosphine ; l'hydrochlorure de tris(2-carboxyéthyl)phosphine ; la tris(3-chlorophényl)phosphine ; la tris(4-chlorophényl)phosphine ; la tris(2-cyanoéthyl)phosphine ; la tris(2,6-diméthoxyphényl)phosphine ; la tris(3-fluorophényl)phosphine ; la tris(4-fluorophényl)phosphine ; la tris(2-furyl)phosphine ; la tris(hydroxyméthyl)phosphine; la tris(2-méthoxyphényl)phosphine ; la tris(3-méthoxyphényl)phosphine ; la tris(4-méthoxyphényl)phosphine ; la tris(3-méthoxypropyl)phosphine ; la tris(4-morpholino)phosphine ; la tris(1-naphtyl)phosphine ; la tris(2-thiényl)phosphine ; la tris(2,4,6-triméthylphényl)phosphine ; la tris(triméthylsilyl)phosphine ; la tri(m-tolyl)phosphine ; la tri(o-tolyl)phosphine ; la tri(p-tolyl)phosphine ; la 2-cyanoéthyldiphénylphosphine ; le 2-dicyclohexylphosphino-2'-méthylbiphényle ; le 2-méthoxyphosphinobenzène ; la benzyldiphénylphosphine ; la bis(2,4,6-triméthylphényl)phosphine ; la bis(2-cyanoéthyl)phosphine ; la bis(3,4,5-triméthoxyphényl)chlorophosphine ; la bis(3,4,5-triméthoxyphényl)phosphine ; la bis(4-fluorophényl)chlorophosphine ; la bis(4-méthoxyphényl)chlorophosphine ; la bis(4-trifluorométhylphényl)chlorophosphine ; la bis(diéthylamino)méthylphosphine ; la bis(diéthylamino)phénylphosphine ; le bis(di-tert-butylphosphino)pentane ; la bis(o-tolyl)chlorophosphine ; la bis(o-tolyl)phosphine ; la dicyclohexylphénylphosphine ; la düsopropylphosphine ; la di-tert-butylhydroxyphosphine ; la di-tert-butylméthylphosphine ; la méthyldichlorophosphine ; le tétrafluoroborate de di-tert-butylméthylphosphine ; l'eicosyl phobane ; les complexes de méthylphosphine borane ; les complexes de phénylphosphine borane ; la tert-butyldiphénylphosphine ; la tert-butylphosphine ; la trüsopropylphosphine ; la tri-n-butylphosphine ; le triphénylphosphine borane ; la tri(3,5-diméthyl-4-méthoxy)phosphine.

Selon un mode de réalisation particulier de l'invention, la ou les phosphines sont choisies parmi les composés de formule (I) suivante : 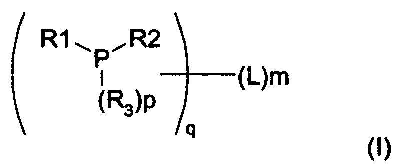 dans laquelle :
- L est un bras de liaison qui représente une liaison covalente ou un radical hydrocarboné bivalent comprenant éventuellement un ou plusieurs hétéroatomes choisis parmi un atome d'oxygène, un atome de soufre, un atome d'azote, un atome de silicium ;
- m est un nombre entier égal à 0 ou à 1 ;
- q est un nombre entier égal à 1 ou à 2 ;
- p est un nombre entier égal à 0 ou à 1 ;
- R₁, R₂ et R₃, identiques ou différents, représentent :
   - un atome d'hydrogène ;
   - un atome d'halogène ;
   - un radical hydroxyle ;
   - un radical carboxy ;
   - un radical hydrocarboné monovalent comprenant éventuellement un ou plusieurs hétéroatomes choisis parmi un atome soufre, un atome d'oxygène, un atome d'azote, un atome de phosphore, un atome de silicium, et éventuellement substitué par un ou plusieurs radicaux choisis parmi :
      - un atome d'halogène,
      - un radical hydroxyle,
      - un radical alcoxy,
      - un radical halogénoalkyle,
      - un radical amino,
      - un radical carboxyle,
      - un radical alcoxycarbonyle,
      - un radical amido,
      - un radical alkylaminocarbonyle,
      - un radical acylamino,
      - un radical mono ou di(alkyl)amino,
      - un radical mono ou di(hydroxyalkyl)amino,
      - un radical N-aryl N-alkyl amino,
      - un cycle aromatique ou hétéroaromatique non substitué ou substitué par un ou plusieurs radicaux choisis parmi un atome d'halogène, un radical hydroxyle, un radical alcoxy, un radical mono ou di(alkyl)amino,
      - un radical cyano,
      - un radical augmentant la solubilité de la phosphine dans l'eau, tel que les radicaux sulfonate, sulfinate, phosphonate, carboxylate,
      - un radical hétérocyclique, aromatique ou non aromatique, non substitué ou substitué ;
   - un radical aryle non substitué ou substitué ;
   - un radical arylalkyle non substitué ou substitué ;
   - un radical arylalkyloxy ;
   - un radical hétérocyclique, aromatique ou non aromatique, non substitué ou substitué ;
   - un radical silyle ;
étant entendu que :
- lorque q = 1, m = 0 et p = 1 ;
- lorsque q = 2, m = 1 et p = 0 ou 1, avec :
   - lorsque p = 0, le bras de liaison L est rattaché à l'atome de phosphore ;
   - lorsque p = 1, le bras de liaison L est rattaché à l'un des radicaux R₁, R₂ et R₃.

Dans toutes les significations précédentes, lorsqu'un radical est substitué, les substituants sont choisis parmi les radicaux halogéno, hydroxy, alkyle, halogénoalkyle, alcoxy, amino, mono ou dialkylamino, mono ou dihydroxyalkylamino, carboxyle. Par exemple, le radical p-méthoxyphényle est un radical aryle substitué.

De préférence, les radicaux R₁, R₂ et R₃ ne représentent pas simultanément un atome d'hydrogène.

Avantageusement, au moins l'un des radicaux R₁, R₂ ou R₃ désigne à titre de radical hydrocarboné un radical alkyle éventuellement substitué.

Selon un mode de réalisation particulier de l'invention, R₁, R₂ et R₃ sont choisis parmi un atome d'hydrogène ; un radical alkyle ; un radical cycloalkyle éventuellement substitué par un ou plusieurs radicaux alkyle ; un radical alcoxy ; un radical alcoxyalkyle ; un radical halogénoalkyle ; un radical cyanoalkyle ; un radical hydroxyalkyle ; un radical carboxyalkyle ; un atome d'halogène ; un radical hydroxyle ; un radical carboxy ; un radical alcényle ; un radical mono ou dialkylamino ; un radical N-aryl N-alkyl aminoalkyle ; un radical aryle éventuellement substitué par un ou plusieurs radicaux choisis parmi un radical alkyle, un radical alcoxy, un radical mono ou dialkylamino, un radical mono ou dialkylaminoalkyle, un radical halogénoalkyle, un radical hydroxyle, un radical carboxy, un atome d'halogène, un radical aryle substitué par un radical mono ou dialkylaminoalkyle ; un radical arylalkyle ; un radical arylalkyloxy ; un radical pyrrolidino ; un radical furyle ; un radical morpholino ; un radical thiényle ; un radical pyridyle ; un radical trialkylsilyle ; un radical alkyle substitué par un radical pyrrolidino, un radical furyle, un radical morpholino ou un radical thiényle.

A titre d'exemple, R₁, R₂ et R₃ peuvent être choisis parmi un atome d'hydrogène ; un radical méthyle ; un radical éthyle ; un radical propyle ; un radical isopropyle ; un radical n-butyle ; un radical isobutyle ; un radical tertio-butyle ; un radical octyle ; un radical cyclohexyle ; un radical cyclopentyle ; un radical méthoxy ; un radical éthoxy ; un radical méthoxypropyle ; un radical chloroéthyle ; un radical cyanoéthyle ; un radical hydroxyméthyle ; un radical hydroxypropyle ; un radical carboxyéthyle ; un atome de chlore ; un radical hydroxyle ; un radical carboxy ; un radical trifluorométhyle ; un radical chlorométhyle ; un radical allyle ; un radical vinyle ; un radical diméthylamino ; un radical diéthylamino ; un radical di(isopropyl)amino ; un radical phényle ; un radical o-tolyle ; un radical m-tolyle ; un radical p-tolyle ; un radical diméthylphényle ; un radical triméthylphényle ; un radical o-méthoxyphényle ; un radical m-méthoxyphényle ; un radical p-méthoxyphényle ; un radical diméthoxyphényle ; un radical triméthoxyphényle ; un radical o-(diméthylamino)phényle ; un radical m-(diméthylamino)phényle ; un radical p-(diméthylamino)phényle ; un radical di(tertio-butyl)phényle ; un radical tri(tertio-butyl)phényle ; un radical trifluorométhylphényle ; un radical bis-(trifluorométhyl)phényle ; un radical o-fluorophényle ; un radical m-fluorophényle ; un radical p-fluorophényle ; un radical o-chlorophényle ; un radical m-chlorophényle ; un radical p-chlorophényle ; un radical o-hydroxyphényle ; un radical m-hydroxyphényle ; un radical p-hydroxyphényle ; un radical 4-(diéthylaminométhyl)phényle ; un radical 3,5-diméthyl-4-méthoxyphényle ; un radical 2-méthyl-biphényle ; un radical benzyle, un radical benzyloxy ; un radical naphtyle ; un radical morpholino ; un radical morpholinométhyle ; un radical pyrrolidino ; un radical furyle ; un radical pyridyle ; un radical thiényle ; un radical triméthylsilyle ; un radical 2-(4-diéthylaminométhylphényl)phényle ; un radical 5-méthyl-2-isopropylcyclohexyle ; un radical N-méthyl N-phényl aminométhyle ; un radical carboxyphényle.

Les phosphines utiles dans le cadre de l'invention peuvent être éventuellement salifiés par des acides minéraux forts tels que par exemple HCl, HBr, H₂SO₄, HBF₄ ou des acides organiques tels que, par exemple, l'acide acétique, lactique, tartrique, citrique ou succinique.

Selon un mode de réalisation particulier de l'invention, la ou les phosphines utiles dans le cadre de l'invention sont choisies parmi les monophosphines. Lorsque la ou les phosphines sont de formule (1), q est alors égal à 1.

A titre d'exemples de monophosphines, on peut citer celles qui ont été énumérées précédemment.

De préférence, les monophosphines utiles dans le cadre de l'invention sont choisies parmi la trihydroxyméthylphosphine ; la trihydroxypropylphosphine ; la bis(hydroxyméthyl)phénylphosphine.

Selon un autre mode de réalisation particulier de l'invention, la ou les phosphines utiles dans le cadre de l'invention sont des diphosphines. Lorsque la ou les phosphines sont de formule (I), q est alors égal à 2.

De préférence, p est égal à 0 et le bras de liaison L est une liaison covalente ou un radical bivalent choisi parmi un radical binaphtylène ; un radical méthylène ; un radical éthyléne ; un radical propylène ; un radical butylène ; un radical pentylène ; un radical hexylène ; un radical phénylène ; un radical méta-diméthylènebenzène ; un radical N-méthyl N'-méthyl hydrazo ; un radical vinylène ; un radical diéthylèneoxy.

A titre d'exemples de diphosphines utiles dans le cadre de l'invention, on peut citer celles qui ont été énumérées précédemment.

Selon un mode de réalisation particulier de l'invention, la ou les phosphines utiles dans le cadre de l'invention sont solubles dans un milieu cosmétiquement acceptable. De préférence, la ou les phosphines utiles dans le cadre de l'invention sont hydrosolubles.

Par « hydrosoluble », on entend dans le cadre de la présente invention toute phosphine dont la solubilité dans l'eau est supérieure à 0,01 % à 20 °C.

La composition de décapage utile dans le cadre de l'invention peut en outre renfermer des agents réducteurs additionnels différents des phosphines tels que ceux qui sont décrits dans la demande de brevet EP 0 943 316. On peut citer notamment les sulfinates, les sucres, les réductones et les acides alpha-oxocarboxyliques tels que l'acide oxalique, l'acide glyoxalique, l'acide pyruvique ou l'acide alpha-cétoglutarique. Lorsqu'ils sont présents, ces agents réducteurs additionnels sont toujours présents en quantité pondérale inférieure à la quantité pondérale des phosphines.

Selon un mode de réalisation particulier de l'invention, la composition de décapage comprend comme unique agent réducteur au moins une phosphine telle que définie précédemment.

La ou les phosphines utiles dans le cadre de l'invention sont en général présentes en quantité comprise entre 0,001 et 30 % en poids environ du poids total de la composition de décapage, de préférence entre 3 et 25 % en poids, et encore plus préférentiellement entre 6 et 20 % en poids.

La composition de décapage utile à l'invention peut également renfermer divers adjuvants utilisés classiquement dans les compositions de décapage, tels que des agents tensioactifs anioniques, cationiques, non-ioniques, amphotères, zwittérioniques ou leurs mélanges, des agents épaississants minéraux ou organiques, et en particulier des épaississants associatifs polymères anioniques, cationiques, non ioniques et amphotères, des polymères conditionneurs non-ioniques, anioniques, amphotères, zwittérioniques, cationiques ou leurs mélanges, de préférence des polymères substantifs cationiques ou amphotères, des agents de pénétration, des agents séquestrants, des parfums, des agents dispersants, des silicones volatiles ou non volatiles, modifiées ou non modifiées, des agents filmogènes, des céramides, des agents conservateurs, des agents opacifiants, des huiles minérales ou végétales, des cires, des vitamines.

Bien entendu, l'homme de l'art veillera à choisir ce ou ces éventuels composés additionnels de manière telle que les propriétés avantageuses attachées intrinsèquement à la composition de décapage utile à l'invention ne soient pas, ou substantiellement pas, altérées par la ou les adjonctions envisagées.

Le milieu cosmétiquement acceptable est généralement constitué par de l'eau ou par un mélange d'eau et d'au moins un solvant organique pour solubiliser les agents réducteurs et / ou les adjuvants qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycol comme le 2-butoxyéthanol, le propylèneglycol, le dipropylène glycol, l'hexylène glycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent alors être présents dans des proportions de préférence comprises entre 0,5 et 20 %, et plus particulièrement entre 2 et 10 % en poids par rapport au poids total de la composition de décapage.

Le pH de la composition de décapage utile à l'invention est de préférence compris entre 2 et 12. En fonction de la phosphine, l'homme du métier définira le pH optimal de la composition de décapage, c'est-à-dire la valeur de pH pour laquelle le décapage obtenu est optimal.

Selon un mode de réalisation particulier de l'invention, la phosphine est la trihydroxyméthylphosphine et le pH de la composition de décapage est d' environ 3.

Selon un autre mode de réalisation particulier de l'invention, la phosphine est la trihydroxypropylphophine et le pH de la composition de décapage est d'environ 9.

Le pH de la composition de décapage utile dans le cadre de l'invention est ajusté à l'aide d'agents acidifiants ou alcalinisants, qui sont en général présents dans la composition dans des proportions de préférence comprises entre 0,01 et 30 % en poids du poids total de la composition de décapage.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, et les acides sulfoniques.

Parmi les agents alcalinisants, on peut citer, à titre d'exemple, l'ammoniaque, les silicates alcalins, les carbonates alcalins, les alcanolamines telles que les mono-, di- et triéthanolamines, le 2-méthyl-2-amino-1-propanol ainsi que leurs dérivés, les hydroxydes de sodium ou de potassium et les composés de formule (II) suivante : 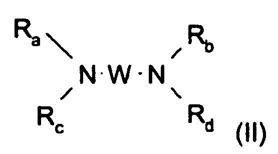 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition de décapage utile à l'invention peut se présenter sous des formes diverses, telles que sous forme de solutions, d'émulsions, de crèmes, de gels, éventuellement pressurisés sous forme de mousses, ou sous toute autre forme appropriée pour réaliser un décapage de la couleur artificielle des fibres kératiniques, et en particulier des fibres kératiniques humaines, telles que les cheveux.

La présente invention a également pour objet un procédé de décapage de la couleur artificielle des fibres kératiniques, caractérisé par le fait que l'on applique sur lesdites fibres kératiniques une composition telle que définie ci-dessus pendant un temps de pose suffisant pour décaper la couleur artificielle des fibres kératiniques.

La température d'application de la composition de décapage est généralement comprise entre 20 et 250 °C, de préférence entre 20 et 80 °C.

Le temps de pose suffisant pour décaper la couleur artificielle des fibres kératiniques est généralement compris entre 1 minutes et 120 minutes, de préférence entre 5 minutes et 60 minutes.

Les conditions d'application, telles que la température d'application et le temps de pose, le pH de la composition de décapage et la quantité de phosphines présentes dans la composition de décapage dépendent de la quantité de colorants artificiels à éliminer et de la nature de la ou des phosphines utilisées.

La présente invention a également pour objet un dispositif à plusieurs compartiments destiné à la teinture puis au décapage de la couleur artificielle des fibres kératiniques, caractérisé par le fait qu'il comprend un premier compartiment renfermant une composition comprenant au moins un précurseur de colorant et / ou un colorant et un deuxième compartiment renfermant une composition de décapage telle que définie ci-dessus.

Selon un mode de réalisation particulier de l'invention, la composition de teinture des fibres kératiniques est une composition de teinture d'oxydation qui comprend au moins une base d'oxydation et / ou au moins un coupleur.

Les bases d'oxydation sont choisies parmi les bases d'oxydation classiquement utilisées en coloration d'oxydation. A titre d'exemples, ces bases d'oxydation sont choisies parmi les para-phénylènediamines, les bases doubles, les para-aminophénols, les ortho-aminophénols, les bases hétérocycliques et leurs sels d'addition.

Parmi les para-phénylènediamines, on peut citer la paraphénylènediamine, la paratoluylènediamine, la 2-chloroparaphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.

Parmi les bases doubles, on peut citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

Parmi les ortho-aminophénols, on peut citer le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

Parmi les bases hétérocycliques, on peut citer les dérivés pyridiniques, et plus particulièrement les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les bases hétérocycliques, on peut également citer les dérivés pyrimidiniques, et plus particulièrement les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino pyrazolo-[1,5-a]-pyrimidine; et leurs sels d'addition et leurs formes tautomères, lorsqu'il existe un équilibre tautomérique et leurs sels d'addition avec un acide.

Parmi les bases hétérocycliques, on peut aussi citer les dérivés pyrazoliques, tels que les composés décrits dans les brevets DE 3 843 892, DE 4 133 957 et demandes de brevet WO 94/08969, WO 94/08970, FR-A-2 733 749 et DE 195 43 988. On peut citer par exemple le 4,5-diamino-1-méthyl-pyrazole, le 3,4-diamino-pyrazole, le 4,5-diamino-1-(4'-chlorobenzyl)-pyrazole, le 4,5-diamino-1,3-diméthyl-pyrazole, le 4,5-diamino-3-méthyl-1-phényl pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, le 4,5-diamino-1-(β-méthoxyéthyl)-pyrazole, et leurs sels d'addition avec un acide.

Les coupleurs sont choisis parmi les coupleurs classiquement utilisés en coloration d'oxydation. A titre d'exemples, ces coupleurs sont choisis parmi les méta-phénylènediamines, les méta-aminophénols, les méta-diphénols, les coupleurs naphtaléniques, les coupleurs hétérocycliques et leurs sels d'addition.

Parmi les coupleurs, on peut notamment citer le 2,4-diamino-1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 1-acétoxy-2-méthyl-naphtalène, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telle que la soude, la potasse, l'ammoniaque, les amines ou les alcanolamines.

La ou les bases d'oxydation sont généralement présentes dans la composition de teinture d'oxydation en quantité comprise entre 0,0005 et 12 % en poids environ du poids total de la composition tinctoriale, de préférence entre 0,005 et 8 % en poids environ.

Le ou les coupleurs sont généralement présents dans la composition de teinture d'oxydation en quantité comprise entre 0,0001 et 15 % en poids environ du poids total de la composition tinctoriale, de préférence entre 0,001 et 10% en poids environ.

Selon un autre mode de réalisation particulier de l'invention, la composition de teinture des fibres kératiniques est une composition de coloration directe qui comprend un ou plusieurs colorants directs pouvant notamment être choisis parmi les colorants nitrés de la série benzénique, les colorants directs azoïques, les colorants directs méthiniques, les colorants directs quinoniques, les colorants directs aziniques, les colorants directs triarylméthaniques, les colorants directs indoaminiques, les colorants directs naturels. Ces colorants directs peuvent être de nature non ionique, anionique ou cationique.

Parmi les colorants directs benzéniques, on peut citer le 1,4-diamino-2-nitrobenzène, le 1-amino-2-nitro-4-(β-hydroxyéthylamino)-benzène, le 1-amino-2-nitro-4-bis(β-hydroxyéthyl)-aminobenzène, le 1,4-bis(β-hydroxyéthylamino)-2-nitro-benzène, le 1-β-hydroxyéthylamino-2-nitro-4-bis-(β-hydroxyéthylamino)-benzène, le 1-β-hydroxyéthylamino-2-nitro-4-amino-benzène, le 1-β-hydroxyéthylamino-2-nitro-4-(éthyl)(β-hydroxyéthyl)-aminobenzène, le 1-amino-3-méthyl-4-β-hydroxyéthylamino-6-nitro-benzène, le 1-amino-2-nitro-4-β-hydroxyéthylamino-5-chloro-benzène, le 1,2-diamino-4-nitro-benzène, le 1-amino-2-β-hydroxyéthylamino-5-nitro-benzène, le 1,2-bis-(β-hydroxyéthylamino)-4-nitro-benzène, le 1-amino-2-[tris-(hydroxyméthyl)-méthylamino]-5-nitrobenzène, le 1-hydroxy-2-amino-5-nitro-benzène, le 1-hydroxy-2-amino-4-nitro-benzène, le 1-hydroxy-3-nitro-4-amino-benzène, le 1-hydroxy-2-amino-4,6-dinitro-benzène, le 1-β-hydroxyéthyloxy-2-β-hydroxyéthylamino-5-nitro-benzène, le 1-méthoxy-2-β-hydroxyéthylamino-5-nitro-benzène, le 1-β-hydroxyéthyloxy-3-méthylamino-4-nitrobenzène, le 1-β,γ-dihydroxypropyloxy-3-méthylamino-4-nitro-benzène, le 1-β-hydroxyéthylamino-4-β,γ-dihydroxypropyloxy-2-nitro-benzène, le 1-β,γ-dihydroxypropylamino-4-trifluorométhyl-2-nitro-benzène, le 1-β-hydroxyéthylamino-4-trifluorométhyl-2-nitro-benzène, le 1-β-hydroxyéthylamino-3-méthyl-2-nitro-benzène, le 1-β-aminoéthylamino-5-méthoxy-2-nitro-benzène, le 1-hydroxy-2-chloro-6-éthylamino-4-nitrobenzène, le 1-hydroxy-2-chloro-6-amino-4-nitro-benzène, le 1-hydroxy-6-[bis-(β-hydroxyéthyl)-amino]-3-nitro-benzène, le 1-β-hydroxyéthylamino-2-nitro-benzène, le 1-hydroxy-4-β-hydroxyéthylamino-3-nitro-benzène.

Parmi les colorants directs azoïques, on peut citer les colorants azoïques cationiques décrits dans les demandes de brevets WO 95/15144, WO-95/01772 et EP 0 714 954 dont le contenu fait partie intégrante de l'invention.

Parmi ces composés, on peut tout particulièrement citer le chlorure de 1,3-diméthyl-2-[[4-(diméthylamino)phényl]azo]-1H-Imidazolium, le chlorure de 1,3-diméthyl-2-[(4-aminophényl)azo]-1H-Imidazolium, le méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants : la 1-N-méthylmorpholiniumpropylamino-4-hydroxyanthraquinone, la 1-aminopropylamino-4-méthylaminoanthraquinone, la 1-aminopropylaminoanthraquinone, la 5-β-hydroxyéthyl-1,4-diaminoanthraquinone, la 2-aminoéthylaminoanthraquinone, la 1,4-bis-(β,γ-dihydroxypropylamino)-anthraquinone.

Parmi les colorants aziniques, on peut citer les composés suivants : Basic Blue 17, Basic Red 2.

Parmi les colorants triarylméthaniques, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques, on peut citer les composés suivants : la 2-β-hydroxyéthlyamino-5-[bis-(β-4'-hydroxyéthyl)amino]anilino-1,4-benzoquinone, la 2-β-hydroxyéthylamino-5-(2'-méthoxy-4'-amino)anilino-1,4-benzoquinone, la 3-N(2'-Chloro-4'-hydroxy)phényl-acétylamino-6-méthoxy-1,4-benzoquinone imine, la 3-N(3'-Chloro-4'-méthylamino)phényl-uréido-6-méthyl-1,4-benzoquinone imine, la 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs sont généralement présents en quantité comprise entre 0,001 et 20% en poids environ du poids total de la composition tinctoriale, et encore plus préférentiellement entre 0,005 et 10% en poids environ.

Selon un autre mode de réalisation particulier de l'invention, la composition de teinture est une composition qui comprend au moins une base d'oxydation, éventuellement au moins un coupleur, et au moins un colorant direct.

Le dispositif à plusieurs compartiments conforme à l'invention peut en outre comprendre un troisième compartiment renfermant une composition oxydante.

Les exemples qui suivent servent à illustrer l'invention sans toutefois présenter un caractère limitatif.

### EXEMPLE 1

Une mèche de cheveux naturels 90 % blancs de 1 g a été préalablement teinte par une teinture Majirouge 6,66 dans les conditions suivantes : 5 g de mélange colorant, réalisé dans les proportions 1 + 1,5 entre le support de teinture et l'oxydant 20 volumes commercialisé par L'OREAL Professionnel, ont été appliqués sur la mèche pendant un temps de pose de 35 minutes à 33 °C. La mèche a ensuite été rincée, lavée avec un shampooing DOP, puis séchée.
La mèche a ensuite été décapée par immersion pendant 30 minutes dans la composition décrite dans le tableau ci-dessous, à une température de 33 °C, à raison de 10 g de composition pour 1 g de cheveux. La mèche a ensuite été rincée, puis shampooinée et séchée.

| | Composition de décapage |
|---|---|
| tri(hydroxyméthyl)phosphine | 5 g |
| lauryl sulfate de sodium | 3 g |
| hydroxypropyl guar | 1 g |
| acide phosphorique | q.s.p pH = 3 |
| eau déminéralisée | q.s.p. 100 g |

Pour vérifier que le décapage des colorants artificiels des fibres kératiniques est bien efficace, on a appliqué sur les fibres kératiniques une solution aqueuse de peroxyde d'hydrogène 20 volumes pendant 3 minutes. La mèche a ensuite été rincée puis shampooinée avec un shampooing DOP et séchée.
Ce traitement n'a pas induit de modification perceptible de la couleur. La composition comprenant la phosphine a bien permis d'éliminer les colorants présents en une étape.
Les valeurs colorimétriques de la mèche séchée ont été prises juste avant le décapage et après le décapage à l'aide d'un spectrocolorimètre Minolta CM-2022. L'écart de couleur ainsi calculé (ΔE) est de 30 ce qui correspond pour ce type de nuance à un décapage important. La légère couleur résiduelle ne gène en rien une recoloration.

### EXEMPLE 2

Une mèche de cheveux naturels 90 % blancs de 1 g a été préalablement teinte par une teinture Majimix 0,600 dans les conditions suivantes : 5 g de mélange colorant, réalisé dans les proportions 1 + 1,5 entre le support de teinture et l'oxydant 20 volumes commercialisé par L'OREAL Professionnel, ont été appliqués sur la mèche pendant un temps de pose de 35 minutes à 33 °C. La mèche a ensuite été rincée, lavée avec un shampooing DOP, puis séchée.
La mèche a ensuite été décapée par immersion pendant 30 minutes dans la composition décrite dans le tableau ci-dessous, à une température de 33 °C, à raison de 10 g de composition pour 1 g de cheveux. La mèche a ensuite été rincée, puis shampooinée et séchée.

| | Composition de décapage |
|---|---|
| tri(hydroxyméthyl)phosphine | 5 g |
| lauryl sulfate de sodium | 3 g |
| hydroxypropyl guar | 1 g |
| acide phosphorique | q.s.p pH = 3 |
| eau déminéralisée | q.s.p. 100 g |

Pour vérifier que le décapage des colorants artificiels des fibres kératiniques est bien efficace, on a appliqué sur les fibres kératiniques une solution aqueuse de peroxyde d'hydrogène 20 volumes pendant 3 minutes. La mèche est rincée puis shampooinée avec un shampooing DOP et séchée.

Ce traitement n'a pas induit de modification perceptible de la couleur. La composition comprenant la phosphine a bien permis d'éliminer les colorants directs présents en une étape.
Les valeurs colorimétriques de la mèche séchée ont été prises juste avant le décapage et après le décapage à l'aide d'un spectrocolorimètre Minolta CM-2022. L'écart de couleur ainsi calculé (ΔE) est de 29,3 ce qui correspond pour ce type de nuance à un décapage important. La légère couleur résiduelle ne gène en rien une recoloration.

### EXEMPLE 3

Une mèche de cheveux naturels 90 % blancs de 1 g a été préalablement teinte par une teinture public Movida 45 dans les conditions suivantes : 5 g de mélange colorant, réalisé dans les conditions recommandées dans le kit, ont été appliqués sur la mèche pendant un temps de pose de 15 minutes à 33 °C. La mèche a ensuite été rincée, lavée avec un shampooing DOP, puis séchée.
La mèche a ensuite été décapée par immersion pendant 30 minutes dans la composition décrite dans le tableau ci-dessous, à une température de 33 °C, à raison de 10 g de composition pour 1 g de cheveux. La mèche a ensuite été rincée, puis shampooinée et séchée.

| | Composition de décapage |
|---|---|
| tri(hydroxyméthyl)phosphine | 5 g |
| lauryl sulfate de sodium | 3 g |
| hydroxypropyl guar | 1 g |
| acide phosphorique | q.s.p pH = 3 |
| eau déminéralisée | q.s.p. 100 g |

Pour vérifier que le décapage des colorants artificiels des fibres kératiniques est bien efficace, on a appliqué sur les fibres kératiniques une solution aqueuse de peroxyde d'hydrogène 20 volumes pendant 3 minutes. La mèche est rincée puis shampooinée avec un shampooing DOP et séchée.
Ce traitement n'a pas induit une modification perceptible de la couleur. La composition comprenant la phosphine a bien permis d'éliminer les colorants présents en une étape.
Les valeurs colorimétriques de la mèche séchée ont été prises juste avant le décapage et après le décapage à l'aide d'un spectrocolorimètre Minolta CM-2022. L'écart de couleur ainsi calculé (ΔE) est de 4,8 ce qui correspond pour ce type de nuance à un décapage notable. La couleur résiduelle n'empêche pas une recoloration.

## Revendications

1. Utilisation pour le décapage de la couleur artificielle des fibres kératiniques d'une composition comprenant, dans un milieu cosmétiquement acceptable, au moins une phosphine ou l'un de ses sels d'addition avec un acide à titre d'agent réducteur majoritaire.

2. Utilisation selon la revendication 1, dans laquelle la ou les phosphines sont choisis parmi les composés de formule (I) suivante : 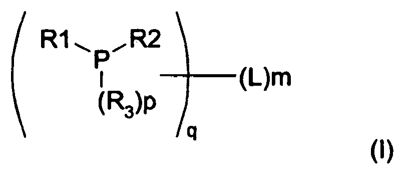 dans laquelle :
• L est un bras de liaison qui représente une liaison covalente ou un radical hydrocarboné bivalent comprenant éventuellement un ou plusieurs hétéroatomes choisis parmi un atome d'oxygène, un atome de soufre, un atome d'azote, un atome de silicium ;
• m est un nombre entier égal à 0 ou à 1 ;
• q est un nombre entier égal à 1 ou à 2 ;
• p est un nombre entier égal à 0 ou à 1 ;
• R₁, R₂ et R₃, identiques ou différents, représentent :
- un atome d'hydrogène ;
- un atome d'halogène ;
- un radical hydroxyle;
- un radical carboxy ;
- un radical hydrocarboné monovalent comprenant éventuellement un ou plusieurs hétéroatomes choisis parmi un atome soufre, un atome d'oxygène, un atome d'azote, un atome de phosphore, un atome de silicium, et éventuellement substitué par un ou plusieurs radicaux choisis parmi :
- un atome d'halogène,
- un radical hydroxyle,
- un radical alcoxy,
- un radical halogénoalkyle,
- un radical amino,
- un radical carboxyle,
- un radical alcoxycarbonyle,
- un radical amido,
- un radical alkylaminocarbonyle,
- un radical acylamino,
- un radical mono ou di(alkyl)amino,
- un radical mono ou di(hydroxyalkyl)amino,
- un radical N-aryl N-alkyl amino,
- un cycle aromatique ou hétéroaromatique non substitué ou substitué par un ou plusieurs radicaux choisis parmi un atome d'halogène, un radical hydroxyle, un radical alcoxy, un radical mono ou di(alkyl)amino,
- un radical cyano,
- un radical augmentant la solubilité de la phosphine dans l'eau,
- un radical hétérocyclique, aromatique ou non aromatique, non substitué ou substitué ;
- un radical aryle non substitué ou substitué ;
- un radical arylalkyle non substitué ou substitué ;
- un radical arylalkyloxy ;
- un radical hétérocyclique, aromatique ou non aromatique, non substitué ou substitué ;
- un radical silyle ;
étant entendu que :
• lorque q = 1, m = 0 et p = 1 ;
• lorsque q = 2, m = 1 et p = 0 ou 1, avec :
- lorsque p = 0, le bras de liaison L est rattaché à l'atome de phosphore ;
- lorsque p = 1, le bras de liaison L est rattaché à l'un des radicaux R₁, R₂ et R₃.

3. Utilisation selon la revendication 2, dans laquelle les radicaux R₁, R₂ et R₃ ne représentent pas simultanément un atome d'hydrogène.

4. Utilisation selon la revendication 2 ou 3, dans laquelle au moins l'un des radicaux R₁, R₂ ou R₃ représente radical hydrocarboné choisi parmi les radicaux alkyle éventuellement substitués.

5. Utilisation selon l'une quelconque des revendications 2 à 4, dans laquelle les radicaux R₁, R₂ et R₃ sont choisis parmi un atome d'hydrogène ; un radical alkyle ; un radical cycloalkyle éventuellement substitué par un ou plusieurs radicaux alkyle ; un radical alcoxy ; un radical alcoxyalkyle ; un radical halogénoalkyle ; un radical cyanoalkyle ; un radical hydroxyalkyle ; un radical carboxyalkyle ; un atome d'halogène ; un radical hydroxyle ; un radical carboxy ; un radical alcényle ; un radical mono ou dialkylamino ; un radical N-aryl N-alkyl aminoalkyle ; un radical aryle éventuellement substitué par un ou plusieurs radicaux choisis parmi un radical alkyle, un radical alcoxy, un radical mono ou dialkylamino, un radical mono ou dialkylaminoalkyle, un radical halogénoalkyle, un radical hydroxyle, un radical carboxy, un atome d'halogène, un radical aryle substitué par un radical mono ou dialkylaminoalkyle ; un radical arylalkyle ; un radical arylalkyloxy ; un radical pyrrolidino ; un radical furyle ; un radical morpholino ; un radical thiényle ; un radical pyridyle; un radical trialkylsilyle ; un radical alkyle substitué par un radical pyrrolidino, un radical furyle, un radical morpholino ou un radical thiényle.

6. Utilisation selon la revendication 5, dans laquelle les radicaux R₁, R₂ et R₃, identiques ou différents, sont choisis parmi un atome d'hydrogène ; un radical méthyle ; un radical éthyle ; un radical propyle ; un radical isopropyle ; un radical n-butyle ; un radical isobutyle ; un radical tertio-butyle ; un radical octyle ; un radical cyclohexyle ; un radical cyclopentyle ; un radical méthoxy; un radical éthoxy; un radical méthoxypropyle ; un radical chloroéthyle ; un radical cyanoéthyle ; un radical hydroxyméthyle ; un radical hydroxypropyle ; un radical carboxyéthyle ; un atome de chlore ; un radical hydroxyle ; un radical carboxy ; un radical trifluorométhyle ; un radical chlorométhyle ; un radical allyle ; un radical vinyle ; un radical diméthylamino; un radical diéthylamino ; un radical di(isopropyl)amino ; un radical phényle ; un radical o-tolyle ; un radical m-tolyle ; un radical p-tolyle ; un radical diméthylphényle ; un radical triméthylphényle ; un radical o-méthoxyphényle ; un radical m-méthoxyphényle; un radical p-méthoxyphényle ; un radical diméthoxyphényle ; un radical triméthoxyphényle ; un radical o-(diméthylamino)phényle ; un radical m-(diméthylamino)phényle ; un radical p-(diméthylamino)phényle ; un radical di(tertio-butyl)phényle ; un radical tri(tertio-butyl)phényle ; un radical trifluorométhylphényle ; un radical bis-(trifluorométhyl)phényle ; un radical o-fluorophényle ; un radical m-fluorophényle ; un radical p-fluorophényle ; un radical o-chlorophényle ; un radical m-chlorophényle ; un radical p-chlorophényle ; un radical o-hydroxyphényle ; un radical m-hydroxyphényle ; un radical p-hydroxyphényle ; un radical 4-(diéthylaminométhyl)phényle ; un radical 3,5-diméthyl-4-méthoxyphényle ; un radical 2-méthyl-biphényle ; un radical benzyle, un radical benzyloxy ; un radical naphtyle ; un radical morpholino ; un radical morpholinométhyle ; un radical pyrrolidino ; un radical furyle ; un radical pyridyle ; un radical thiényle ; un radical triméthylsilyle ; un radical 2-(4-diéthylaminométhylphényl)phényle ; un radical 5-méthyl-2-isopropylcyclohexyle ; un radical N-méthyl N-phényl aminométhyle ; un radical carboxyphényle.

7. Utilisation selon l'une quelconque des revendications précédentes, dans laquelle la ou les phosphines sont choisies parmi les monophosphines.

8. Utilisation selon l'une quelconque des revendications 2 à 7, dans laquelle la ou les phosphines de formule (I) sont telles que q est égal à 1.

9. Utilisation selon l'une quelconque des revendications précédentes, dans laquelle la ou les phosphines sont choisies parmi la tri(hydroxyméthyl)phosphine, la tri(hydroxypropyl)phosphine, la bis(hydroxyméthyl)(phényl)phosphine ; l'allyldiphénylphosphine ; la benzyldiphénylphosphine ; la bis(3,4,5-triméthoxyphényl)chlorophosphine ; la bis(3,4,5-triméthoxyphényl)phosphine ; la benzyloxy(diisopropylamino)méthylphosphine ; la bis(diisopropylamino)chlorophosphine ; la bis(2-cyanoéthyl)phosphine ; la bis(3,5-di-tert-butytphényl)chlorophosphine ; la bis(3,5-di-tert-butylphényl)phosphine ; la bis(diéthylamino)méthylphosphine ; la bis(diéthylamino)chlorophosphine ; la bis(diéthylamino)phénylphosphine ; la bis(3,5-diméthyl-4-méthoxyphényl)chlorophosphine ; la bis(3,5-diméthyl-4-méthoxyphényl)phosphine ; la bis(3,5-diméthylphényl)chlorophosphine ; la bis(3,5-diméthylphényl)diéthylaminophosphine ; la bis(3,5-diméthylphényl)phosphine ; la bis(3,5-ditrifluorométhylphényl)chlorophosphine ; la bis(3,5-ditrifluorométhylphényl)phosphine ; la bis(4-fluorophényl)chlorophosphine ; la bis(2-furyl)chlorophosphine ; la bis(2-furyl)phosphine ; la bis(hydroxyméthyl)phénylphosphine , la bis(4-méthoxyphényl)phénylphosphine ; la bis(3,5-diméthylphényl)phosphine ; la bis(3,5-di-tert-butylphényl)chlorophosphine ; la bis(3,5-di-tert-butylphényl)phosphine ; la bis(3,5-ditrifluorométhylphényl)chlorophosphine ; la bis(3,5-ditrifluorométhylphényl)phosphine ; la bis(4-fluorophényl)chlorophosphine ; la bis(4-méthoxyphényl)chlorophosphine ; la bis(4-méthoxyphényl)phenylphosphine ; la bis(4-méthylphényl)chlorophosphine ; la bis(4-méthylphényl)phosphine; la bis(4-trifluorométhylphényl)chlorophosphine ; la bis(4-trifluorométhylphényl)phosphine ; la bis(diéthylamino)méthylphosphine ; la bis(diéthylamino)phénylphosphine ; la bis(hydroxyméthyl)phénylphosphine ; la bis(o-tolyl)chlorophosphine; la bis(o-tolyl)phosphine ; la bis(pyrrolidino)méthylphosphine ; la butyldichlorophosphine ; la butyldiphénylphosphine ; la tert-butyldiphénylphosphine ; la cyclohexyl(diéthylamino)chlorophosphine ; la cyclohexyl(diméthylamino)chlorophosphine ; la cyclohexyldichlorophosphine ; la cyclohexyldiphénylphosphine ; la 2-chloroéthyldiphénylphosphine ; la 2-(dicyclohexylphosphino)biphényle ; la 2-dicyclohexylphosphino-2'-(N,N-diméthylamino)biphényle ; la diéthylaminodiéthylphosphine ; la diméthylaminodichlorophosphine ; la (4-diméthylaminophényl)diphénylphosphine ; la N-[(diphénylphosphinyl)méthyl]-N-méthylaniline ; l'acide o-diphénylphosphinobenzoïque ; la 2-méthoxy(dichlorophosphino)benzène ; la 4-méthoxyphényl(diéthylamino)chlorophosphine ; la 4-méthoxyphényl(diméthylamino)chlorophosphine ; la (2-méthoxyphényl)méthylphénylphosphine ; la 2-méthoxyphosphinobenzène ; la (5-méthyl-2-isopropylcyclohexyl)diphénylphosphine ; la triphénylphosphine ; la diallylphénylphosphine ; la dibenzylphosphine ; la dibutylphénylphosphine ; la dibutylphosphine ; la dicyclohexylchlorophosphine ; la dicyclohexylphénylphosphine ; la dicyclohexylphosphine ; la diéthylchlorophosphine ; la diéthylphénylphosphine ; la diéthylphosphine ; la diisobutylphosphine; la diisopropylchlorophosphine ; la diisopropylphosphine ; la diméthyl(phényl)phosphine ; la diméthyl(triméthylsily)phosphine ; la diméthylchlorophosphine ; la diphényl(o-tolyl)phosphine ; la diphényl(p-tolyl)phosphine ; la diphényl(triméthylsilyl)phosphine ; la diphénylchlorophosphine ; la diphénylphosphine ; la diphénylpropylphosphine ; la diphénylvinylphosphine ; la di-tert-butylchlorophosphine ; la di-tert-butylhydroxyphosphine ; la di-tert-butylméthylphosphine ; la di-tert-butylphénylphosphine ; la di-tert-butylphosphine ; la divinylphénylphosphine ; l'éthyldichlorophosphine ; l'éthyidiphénylphosphine ; l'isopropyldichlorophosphine ; la méthoxydiéthoxyphosphine ; la méthyldichlorophosphine ; la méthyldiphénylphosphine ; la méthylphénylchlorophosphine ; la phénylphosphine ; la propyldichlorophosphine ; la tert-butylbis(triméthylsilyl)phosphine ; la tert-butyldichlorophosphine ; la tert-butyldiéthylphosphine ; la tert-butyldiphénylphosphine ; la tert-butylphosphine ; la tri(m-tolyl)phosphine ; la tri(o-tolyl)phosphine ; la tri(p-tolyl)phosphine ; la tricyclohexylphosphine ; la tricyclopentylphosphine ; la triéthylphosphine ; la triisobutylphosphine ; la triisopropylphosphine ; la triméthylphosphine ; la tri-n-butylphosphine ; la tri-n-octylphosphine ; la tripropylphosphine ; la tris(1-naphthyl)phosphine ; la tris(2,4,6-triméthylphényl)phosphine ; la tris(2,6-diméthoxyphényl)phosphine ; la tris(2-carboxyéthyl)phosphine ; la tris(2-cyanoéthyl)phosphine ; la tris(2-furyl)phosphine ; la tris(2-méthoxyphényl)phosphine ; la tris(2-thiényl)phosphine ; la tris(3,5-diméthyl-4-méthoxy)phosphine ; la tris(3-chlorophényl)phosphine ; la tris(3-fluorophényl)phosphine ; la tris(3-méthoxyphényl)phosphine ; la tris(3-méthoxypropyl)phosphine ; la tris(4-chlorophényl)phosphine ; la tris(4-fluorophényl)phosphine ; la tris(4-méthoxyphényl)phosphine ; la tris(4-morpholino)phosphine ; la tris(hydroxyméthyl)phosphine ; la tris(triméthylsilyl)phosphine ; la tris[3,5-bis(trifluorométhyl)phényl]phosphine ; la tri-tert-butylphosphine ; la 2-cyanoéthyldiphénylphosphine ; le 2-dicyclohexylphosphino-2'-méthylbiphényle ; la bis(2,4,6-triméthylphényl)phosphine ; le 2-(di-tert-butylphosphino)biphényle.

10. Utilisation selon la revendication 9, dans laquelle la ou les phosphines sont choisies parmi la trihydroxyméthylphosphine ; la trihydroxypropylphosphine ; la bis(hydroxyméthyl)phénylphosphine.

11. Utilisation selon l'une quelconque des revendications 1 à 6, dans laquelle la ou les phosphines sont choisies parmi les diphosphines.

12. Utilisation selon l'une quelconque des revendications 2 à 6 et 11, dans laquelle la ou les phosphines de formule (I) sont telles que q est égal à 2.

13. Utilisation selon la revendication 12, dans laquelle p est égal à 0 et le bras de liaison L est une liaison covalente ou un radical bivalent choisi parmi un radical binaphtylène ; un radical méthylène ; un radical éthylène ; un radical propylène ; un radical butylène ; un radical pentylène ; un radical hexylène ; un radical phénylène ; un radical méta-diméthylènebenzène ; un radical N-méthyl N'-méthyl hydrazo ; un radical vinylène ; un radical diéthylèneoxy.

14. Utilisation selon l'une quelconque des revendications 1 à 6 et 11 à 13, dans laquelle la ou les phosphines sont choisies parmi le 2,2'-bis(dicyclohexylphosphino)-1,1'-binaphtyle ; le 2,2'-bis[bis(3,5-diméthylphénylphosphino)]-1,1'-binaphtyle ; le 1,4-bis[bis(3,5-diméthylphényl)phosphino]butane ; le 1,2-bis[bis(3,5-diméthylphényl)phosphino]éthane ; le bis[bis(3,5-diméthylphényl)phosphino]méthane ; le 1,5-bis[bis(3,5-diméthylphényl)phosphino]pentane ; le 1,3-bis[bis(3,5-diméthylphényl)phosphino]propane ; le 2,2'-bis[bis(3,5-ditrifluorométhylphényl)phosphino]-1,1'-binaphtyle ; le 1,4-bis[bis(3,5-ditrifluorométhylphényl)phosphino]butane ; le 1,2-bis[bis(3,5-ditrifluorométhylphényl)phosphino]éthane ; le bis[bis(3,5-ditrifluorométhylphényl)phosphino]méthane ; le 1,5-bis[bis(3,5-ditrifluorométhylphényl)phosphino]pentane ; le 1,3-bis[bis(3,5-ditrifluorométhylphényl)phosphino]propane ; le 1,2-bis(di-tert-butylphosphino)benzène ; le 1,4-bis(di-tert-butylphosphino)butane ; le 1,2-bis(di-tert-butylphosphino)éthane ; le 1,3-bis(di-tert-butylphosphinométhyl)benzène ; le 1,3-bis(di-tert-butylphosphino)propane ; le 1,2-bis(dichlorophosphino)benzène ; le 1,3-bis(dichlorophosphino)benzène ; le 1,4-bis(dichlorophosphino)benzène ; le 1,4-bis(dichlorophosphino)butane ; la 1,2-bis(dichlorophosphino)-1,2-diméthylhydrazine ; le 1,2-bis(dichlorophosphino)éthane ; le bis(dichlorophosphino)méthane ; le 1,3-bis(dichlorophosphino)propane ; le 1,2-bis(dicyclohexylphosphino)benzène ; le 2,2'-bis(dicyclohexylphosphinor)-1,1'-binaphtyle ; le 1,4-bis(dicyclohexylphosphino)butane ; le (2R,3R)-bis(dicyclohexylphosphino)butane ; le (2S,3S)-bis(dicyclohexylphosphino)butane ; le 1,2-bis(dicyclohexylphosphino)éthane ; le bis(dicyclohexylphosphino)méthane ; le 1,3-bis(dicyclohexylphosphino)propane ; le bis[2-(4-diéthylaminométhylphényl)-phénylphosphino]éthyl éther ; le 1,2-bis(diéthylphosphino)éthane ; le 1,2-bis(diméthylphosphino)benzène ; le 1,4-bis(diméthylphosphino)butane ; le 1,2-bis(diméthylphosphino)éthane ; le bis(diméthylphosphino)méthane ; le 1,3-bis(diméthylphosphino)propane ; le 1,2-bis(diphénylphosphino)benzène ; le 1,3-bis(diphénylphosphino)benzène ; le 1,4-bis(diphénylphosphino)benzène ; le 2,2'-bis(diphénylphosphino)-1,1'-binaphtyle ; le 1,4-bis(diphénylphosphino)butane ; le 1,2-bis(diphénylphosphino)éthane ; le cis-1,2-bis(diphénylphosphino)éthylène ; le trans-1,2-bis(diphénylphosphino)éthylène ; le bis(2-diphénylphosphino)éthyl éther ; le 1,6-bis(diphénylphosphino)hexane ; le bis(diphénylphosphino)méthane ; le 1,5-bis(diphénylphosphino)pentane ; le 1 ,3-bis(diphénylphosphino)propane ; le 1,2-bis(ditrifluorométhylphosphino)éthane ; le 1,2-bis[(2-méthoxyphényl)phénylphosphino]éthane ; le 1,2-bis(phénylphosphino)éthane ; le 1,3-bis(phénylphosphino)propane ; le bis-2-[(phényl)(3-pyridyl)phosphinoéthyl] éther; le 1,2-bis(phosphino)benzène ; le 1,2-bis(phosphino)éthane ; le bis(phosphino)méthane ; le 1,2-bis(trifluorométhyl)phosphino)éthane ; le bis(di-tert-butylphosphino)pentane ; la tétraphénylbiphosphine.

15. Utilisation selon l'une quelconque des revendications précédentes, dans laquelle la ou les phosphines sont des composés solubles dans un milieu cosmétiquement acceptable.

16. Utilisation selon la revendication 15, dans laquelle la ou les phosphines sont hydrosolubles.

17. Utilisation selon l'une quelconque des revendications précédentes, dans laquelle la composition comprend comme unique agent réducteur la ou les phosphines.

18. Utilisation selon l'une quelconque des revendications précédentes, dans laquelle la ou les phosphines sont présentes en quantité comprise entre 0,001 et 30 % en poids environ du poids total de la composition.

19. Utilisation selon l'une quelconque des revendications précédentes, dans laquelle le pH de la composition de décapage est compris entre 2 et 12.

20. Utilisation selon la revendication 19, dans laquelle la phosphine est la trihydroxyméthylphosphine et le pH de la composition de décapage est d' environ 3.

21. Utilisation selon la revendication 19, dans laquelle la phosphine est la trihydroxypropylphophine et le pH de la composition de décapage est d'environ 9.

22. Procédé de décapage de la couleur artificielle des fibres kératiniques, **caractérisé par le fait que** l'on applique sur lesdites fibres kératiniques une composition telle que définie à l'une quelconque des revendications 1 à 21 pendant un temps de pose suffisant pour décaper la couleur artificielle des fibres kératiniques.

23. Dispositif à plusieurs compartiments destiné à la teinture puis au décapage de la couleur artificielle des fibres kératiniques, **caractérisé par le fait qu**'il comprend un premier compartiment renfermant une composition comprenant au moins un précurseur de colorant et / ou un colorant et un deuxième compartiment renfermant une composition de décapage telle que définie à l'une quelconque des revendications 1 à 21.

24. Dispositif selon la revendication 23, dans lequel le ou les précurseurs de colorant sont choisis parmi les bases d'oxydation et / ou les coupleurs.

25. Dispositif selon la revendication 23 ou 24, dans lequel le ou les colorants sont choisis parmi les colorants directs.

26. Dispositif selon l'une quelconque des revendications 23 à 25, comprenant de plus un troisième compartiment renfermant une composition oxydante.

## Claims

1. Use, for stripping artificial colour from keratin fibres, of a composition comprising, in a cosmetically acceptable medium, at least one phosphine or an acid-addition salt thereof as major reducing agent.

2. Use according to Claim 1, in which the phosphine(s) is (are) chosen from the compounds of formula (I) below: 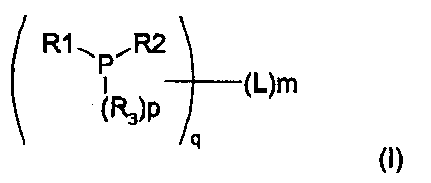 in which:
• L is a linker that represents a covalent bond or a divalent hydrocarbon-based radical optionally comprising one or more hetero atoms chosen from an oxygen atom, a sulfur atom, a nitrogen atom and a silicon atom;
• m is an integer equal to 0 or 1;
• q is an integer equal to 1 or 2;
• p is an integer equal to 0 or 1;
• R₁, R₂ and R₃, which may be identical or different, represent:
- a hydrogen atom;
- a halogen atom;
- a hydroxyl radical;
- a carboxyl radical;
- a monovalent hydrocarbon-based radical optionally comprising one or more hetero atoms chosen from a sulfur atom, an oxygen atom, a nitrogen atom, a phosphorus atom and a silicon atom, optionally substituted with one or more radicals chosen from:
- a halogen atom,
- a hydroxyl radical,
- an alkoxy radical,
- a haloalkyl radical,
- an amino radical,
- a carboxyl radical,
- an alkoxycarbonyl radical,
- an amido radical,
- an alkylaminocarbonyl radical,
- an acylamino radical,
- a mono- or di(alkyl)amino radical,
- a mono- or di(hydroxyalkyl)amino radical,
- an N-aryl-N-alkylamino radical,
- an aromatic or heteroaromatic ring, which is unsubstituted or substituted with one or more radicals chosen from a halogen atom, a hydroxyl radical, an alkoxy radical and a mono- or di(alkyl)amino radical,
- a cyano radical,
- a radical that increases the solubility of the phosphine in water,
- a substituted or unsubstituted, aromatic or non-aromatic heterocyclic radical;
- a substituted or unsubstituted aryl radical;
- a substituted or unsubstituted arylalkyl radical;
- an arylalkyloxy radical;
- a substituted or unsubstituted, aromatic or non-aromatic heterocyclic radical;
- a silyl radical;
it being understood that:
• when q = 1, m = 0 and p = 1;
• when q = 2, m = 1 and p = 0 or 1, with:
- when p = 0, the linker L is attached to the phosphorus atom;
- when p = 1, the linker L is attached to one of the radicals R₁, R₂ and R₃.

3. Use according to Claim 2, in which the radicals R₁, R₂ and R₃ do not simultaneously represent a hydrogen atom.

4. Use according to Claim 2 or 3, in which at least one of the radicals R₁, R₂ and R₃ represents a hydrocarbon-based radical chosen from optionally substituted alkyl radicals.

5. Use according to any one of Claims 2 to 4, in which the radicals R₁, R₂ and R₃ are chosen from a hydrogen atom; an alkyl radical; a cycloalkyl radical optionally substituted with one or more alkyl radicals; an alkoxy radical; an alkoxyalkyl radical; a haloalkyl radical; a cyanoalkyl radical; a hydroxyalkyl radical; a carboxyalkyl radical; a halogen atom; a hydroxyl radical; a carboxyl radical; an alkenyl radical; a mono- or dialkylamino radical; an N-aryl-N-alkylaminoalkyl radical; an aryl radical optionally substituted with one or more radicals chosen from an alkyl radical, an alkoxy radical, a mono- or dialkylamino radical, a mono- or dialkylaminoalkyl radical, a haloalkyl radical, a hydroxyl radical, a carboxyl radical, a halogen atom, an aryl radical substituted with a mono- or dialkylaminoalkyl radical; an arylalkyl radical; an arylalkyloxy radical; a pyrrolidino radical; a furyl radical; a morpholino radical; a thienyl radical; a pyridyl radical; a trialkylsilyl radical; an alkyl radical substituted with a pyrrolidino radical, a furyl radical, a morpholino radical or a thienyl radical.

6. Use according to Claim 5, in which the radicals R₁, R₂ and R₃, which may be identical or different, are chosen from a hydrogen atom; a methyl radical; an ethyl radical; a propyl radical; an isopropyl radical; an n-butyl radical; an isobutyl radical; a tert-butyl radical; an octyl radical; a cyclohexyl radical; a cyclopentyl radical; a methoxy radical; an ethoxy radical; a methoxypropyl radical; a chloroethyl radical; a cyanoethyl radical; a hydroxymethyl radical; a hydroxypropyl radical; a carboxyethyl radical; a chlorine atom; a hydroxyl radical; a carboxyl radical; a trifluoromethyl radical; a chloromethyl radical; an allyl radical; a vinyl radical; a dimethylamino radical; a diethylamino radical; a di(isopropyl)amino radical; a phenyl radical; an o-tolyl radical; an m-tolyl radical; a p-tolyl radical; a dimethylphenyl radical; a trimethylphenyl radical; an o-methoxyphenyl radical; an m-methoxyphenyl radical; a p-methoxyphenyl radical; a dimethoxyphenyl radical; a trimethoxyphenyl radical; an o-(dimethylamino)phenyl radical; an m-(dimethylamino)phenyl radical; a p-(dimethylamino)phenyl radical; a di(tert-butyl)phenyl radical; a tri(tert-butyl)phenyl radical; a trifluoromethylphenyl radical; a bis(trifluoromethyl)phenyl radical; an o-fluorophenyl radical; an m-fluorophenyl radical; a p-fluorophenyl radical; an o-chlorophenyl radical; an m-chlorophenyl radical; a p-chlorophenyl radical; an o-hydroxyphenyl radical; an m-hydroxyphenyl radical; a p-hydroxyphenyl radical; a 4-(diethylaminomethyl)phenyl radical; a 3,5-dimethyl-4-methoxyphenyl radical; a 2-methylbiphenyl radical; a benzyl radical; a benzyloxy radical; a naphthyl radical; a morpholino radical; a morpholinomethyl radical; a pyrrolidino radical; a furyl radical; a pyridyl radical; a thienyl radical; a trimethylsilyl radical; a 2-(4-diethylaminomethylphenyl)phenyl radical; a 5-methyl-2-isopropylcyclohexyl radical; an N-methyl-N-phenylaminomethyl radical; a carboxyphenyl radical.

7. Use according to any one of the preceding claims, in which the phosphine(s) is (are) chosen from monophosphines.

8. Use according to any one of Claims 2 to 7, in which the phosphine(s) of formula (I) is (are) such that q is equal to 1.

9. Use according to any one of the preceding claims, in which the phosphine(s) is (are) chosen from tri(hydroxymethyl)phosphine; tri(hydroxypropyl)phosphine; bis(hydroxymethyl)(phenyl)phosphine; allyldiphenylphosphine; benzyldiphenylphosphine; bis(3,4,5-trimethoxyphenyl)chlorophosphine; bis(3,4,5-trimethoxyphenyl)phosphine; benzyloxy(diisopropylamino)methylphosphine; bis(diisopropylamino)chlorophosphine; bis(2-cyanoethyl)phosphine; bis(3,5-di-tert-butylphenyl)chlorophosphine; bis(3,5-di-tert-butylphenyl)phosphine; bis(diethylamino)methylphosphine; bis(diethylamino)chlorophosphine; bis(diethylamino)phenylphosphine; bis (3,5-dimethyl-4-methoxyphenyl)chlorophosphine; bis (3,5-dimethyl-4-methoxyphenyl)phosphine; bis (3,5-dimethylphenyl)chlorophosphine; bis (3,5-dimethylphenyl)diethylaminophosphine; bis (3,5-dimethylphenyl)phosphine; bis (3,5-ditrifluoromethylphenyl)chlorophosphine; bis (3,5-ditrifluoromethylphenyl) phosphine; bis(4-fluorophenyl)chlorophosphine; bis(2-furyl)chlorophosphine; bis(2-furyl)phosphine; bis(hydroxymethyl)phenylphosphine; bis(4-methoxyphenyl)phenylphosphine; bis(3,5-dimethylphenyl)-phosphine; bis(3,5-di-tert-butylphenyl)chlorophosphine; bis(3,5-di-tert-butylphenyl)phosphine; bis(3,5-ditrifluoromethylphenyl)chlorophosphine; bis(3,5-ditrifluoromethylphenyl)phosphine; bis(4-fluorophenyl)-chlorophosphine; bis(4-methoxyphenyl)chlorophosphine; bis(4-methoxyphenyl)phenylphosphine; bis(4-methylphenyl)chlorophosphine; bis(4-methylphenyl)phosphine; bis(4-trifluoromethylphenyl)chlorophosphine; bis(4-trifluoromethylphenyl)phosphine; bis(diethylamino)methylphosphine; bis(diethylamino)phenylphosphine; bis(hydroxymethyl)phenylphosphine; bis(o-tolyl)chlorophosphine; bis(o-tolyl)phosphine; bis(pyrrolidino)methylphosphine; butyldichlorophosphine; butyldiphenylphosphine; tert-butyldiphenylphosphine; cyclohexyl-(diethylamino)chlorophosphine; cyclohexyl(dimethylamino)chlorophosphine; cyclohexyldichlorophosphine; cyclohexyldiphenylphosphine; 2-chloroethyldiphenylphosphine; 2-(dicyclohexylphosphino)biphenyl; 2-dicyclohexylphosphino-2'-(N,N-dimethylamino)biphenyl; diethylaminodiethylphosphine; dimethylaminodichlorophosphine; (4-dimethylaminophenyl)diphenylphosphine; N-[(diphenylphosphinyl)methyl]-N-methylaniline; o-diphenylphosphinobenzoic acid; 2-methoxy(dichlorophosphino)benzene; 4-methoxyphenyl(diethylamino)chlorophosphine; 4-methoxyphenyl(dimethylamino)chlorophosphine; (2-methoxyphenyl)methylphenylphosphine; 2-methoxyphosphinobenzene; (5-methyl-2-isopropylcyclohexyl)diphenylphosphine; triphenylphosphine; diallylphenylphosphine; dibenzylphosphine; dibutylphenylphosphine; dibutylphosphine; dicyclohexylchlorophosphine; dicyclohexylphenylphosphine; dicyclohexylphosphine; diethylchlorophosphine; diethylphenylphosphine; diethylphosphine; diisobutylphosphine; diisopropylchlorophosphine; diisopropylphosphine; dimethyl(phenyl)phosphine; dimethyl(trimethylsilyl)phosphine; dimethylchlorophosphine; diphenyl(o-tolyl)phosphine; diphenyl(p-tolyl)phosphine; diphenyl(trimethylsilyl)phosphine; diphenylchlorophosphine; diphenylphosphine; diphenylpropylphosphine; diphenylvinylphosphine; di-tert-butylchlorophosphine; di-tert-butylhydroxyphosphine; di-tert-butylmethylphosphine; di-tert-butylphenylphosphine; di-tert-butylphosphine; divinylphenylphosphine; ethyldichlorophosphine; ethyldiphenylphosphine; isopropyldichlorophosphine; methoxydiethoxyphosphine; methyldichlorophosphine; methyldiphenylphosphine; methylphenylchlorophosphine; phenylphosphine; propyldichlorophosphine; tert-butylbis(trimethylsilyl)phosphine; tert-butyldichlorophosphine; tert-butyldiethylphosphine; tert-butyldiphenylphosphine; tert-butylphosphine; tri(m-tolyl)phosphine; tri(o-tolyl)phosphine; tri(p-tolyl)phosphine; tricyclohexylphosphine; tricyclopentylphosphine; triethylphosphine; triisobutylphosphine; triisopropylphosphine; trimethylphosphine; tri-n-butylphosphine; tri-n-octylphosphine; tripropylphosphine; tris (1-naphthyl)phosphine; tris (2,4,6-trimethylphenyl)phosphine; tris (2,6-dimethoxyphenyl)phosphine; tris(2-carboxyethyl)-phosphine; tris(2-cyanoethyl)phosphine; tris(2-furyl)phosphine; tris(2-methoxyphenyl)phosphine; tris(2-thienyl)phosphine; tris(3,5-dimethyl-4-methoxy)phosphine; tris(3-chlorophenyl)phosphine; tris (3-fluorophenyl) phosphine; tris(3-methoxyphenyl)phosphine; tris(3-methoxypropyl)phosphine; tris(4-chlorophenyl)-phosphine; tris(4-fluorophenyl)phosphine; tris(4-methoxyphenyl)phosphine; tris(4-morpholino)phosphine; tris(hydroxymethyl)phosphine; tris(trimethylsilyl)phosphine; tris[3,5-bis(trifluoromethyl)phenyl]phosphine; tri-tert-butylphosphine; 2-cyanoethyldiphenylphosphine; 2-dicyclohexylphosphino-2'-methylbiphenyl; bis(2,4,6-trimethylphenyl)phosphine; 2-(di-tert-butylphosphino)biphenyl.

10. Use according to Claim 9, in which the phosphine(s) is (are) chosen from trihydroxymethylphosphine; trihydroxypropylphosphine; bis(hydroxymethyl)-phenylphosphine.

11. Use according to any one of Claims 1 to 6, in which the phosphine(s) is (are) chosen from diphosphines.

12. Use according to any one of Claims 2 to 6 and 11, in which the phosphine(s) of formula (I) is (are) such that q is equal to 2.

13. Use according to Claim 12, in which p is equal to 0 and the linker L is a covalent bond or a divalent radical chosen from a binaphthylene radical; a methylene radical; an ethylene radical; a propylene radical; a butylene radical; a pentylene radical; a hexylene radical; a phenylene radical; a meta-dimethylenebenzene radical; an N-methyl-N'-methylhydrazo radical; a vinylene radical; a diethyleneoxy radical.

14. Use according to any one of Claims 1 to 6 and 11 to 13, in which the phosphine(s) is (are) chosen from 2,2'-bis(dicyclohexylphosphino)-1,1'-binaphthyl; 2,2'-bis[bis(3,5-dimethylphenylphosphino)]-1,1'-binaphthyl; 1,4-bis[bis(3,5-dimethylphenyl)phosphino]butane; 1,2-bis[bis(3,5-dimethylphenyl)phosphino]ethane; bis[bis(3,5-dimethylphenyl)phosphino]methane; 1,5-bis[bis(3,5-dimethylphenyl)phosphino]pentane; 1,3-bis[bis(3,5-dimethylphenyl)phosphino]propane; 2,2'-bis[bis(3,5-ditrifluoromethylphenyl)-phosphino]-1,1'-binaphthyl; 1,4-bis[bis(3,5-ditrifluoromethylphenyl)phosphino]butane; 1,2-bis[bis(3,5-ditrifluoromethylphenyl)-phosphino]ethane; bis[bis(3,5-ditrifluoromethylphenyl)phosphino]methane; 1,5-bis[bis(3,5-ditrifluoromethylphenyl)phosphino]pentane; 1,3-bis[bis(3,5-ditrifluoromethylphenyl)phosphino]-propane; 1,2-bis(di-tert-butylphosphino)benzene; 1,4-bis(di-tert-butylphosphino)butane; 1,2-bis(di-tert-butylphosphino)ethane; 1,3-bis(di-tert-butylphosphinomethyl)benzene; 1,3-bis(di-tert-butylphosphino)propane; 1,2-bis(dichlorophosphino)benzene; 1,3-bis(dichlorophosphino)benzene; 1,4-bis(dichlorophosphino)-benzene; 1,4-bis(dichlorophosphino)butane; 1,2-bis(dichlorophosphino)-1,2-dimethylhydrazine; 1,2-bis(dichlorophosphino)ethane; bis(dichlorophosphino)methane; 1,3-bis(dichlorophosphino)propane; 1,2-bis(dicyclohexylphosphino)benzene; 2,2'-bis(dicyclohexylphosphino)-1,1'-binaphthyl; 1,4-bis(dicyclohexylphosphino)butane; (2R,3R)-bis(dicyclohexylphosphino)butane; (2S, 3S)-bis(dicyclohexylphosphino)butane; 1,2-bis(dicyclohexylphosphino)ethane; bis(dicyclohexylphosphino)methane; 1,3-bis(dicyclohexylphosphino)propane; bis[2-(4-diethylaminomethylphenyl)phenylphosphino]ethyl ether; 1,2-bis(diethylphosphino)ethane; 1,2-bis(dimethylphosphino)benzene; 1,4-bis(dimethylphosphino)butane; 1,2-bis(dimethylphosphino)ethane; bis(dimethylphosphino)methane; 1,3-bis(dimethylphosphino)propane; 1,2-bis(diphenylphosphino)benzene; 1,3-bis(diphenylphosphino)benzene; 1,4-bis-(diphenylphosphino)benzene; 2,2'-bis(diphenylphosphino)-1,1'-binaphthyl; 1,4-bis(diphenylphosphino)butane; 1,2-bis(diphenylphosphino)-ethane; cis-1,2-bis(diphenylphosphino)ethylene; trans-1,2-bis(diphenylphosphino)ethylene; bis(2-diphenylphosphino)ethyl ether; 1,6-bis(diphenylphosphino)hexane; bis(diphenylphosphino)methane; 1,5-bis(diphenylphosphino)pentane; 1,3-bis(diphenylphosphino)propane; 1,2-bis(ditrifluoromethylphosphino)ethane; 1,2-bis[(2-methoxyphenyl)phenylphosphino]ethane; 1,2-bis(phenylphosphino)ethane; 1,3-bis(phenylphosphino)propane; bis-2-[(phenyl)(3-pyridyl)phosphinoethyl] ether; 1,2-bis(phosphino)benzene; 1,2-bis(phosphino)ethane; bis(phosphino)methane; 1,2-bis(trifluoromethyl)-phosphino)ethane; bis(di-tert-butylphosphino)-pentane; tetraphenylbiphosphine.

15. Use according to any one of the preceding claims, in which the phosphine(s) is (are) compounds that are soluble in a cosmetically acceptable medium.

16. Use according to Claim 15, in which the phosphine(s) is (are) water-soluble.

17. Use according to any one of the preceding claims, in which the composition comprises the phosphine(s) as sole reducing agent.

18. Use according to any one of the preceding claims, in which the phosphine(s) is (are) present in an amount of between 0.001% and 30% by weight approximately relative to the total weight of the composition.

19. Use according to any one of the preceding claims, in which the pH of the stripping composition is between 2 and 12.

20. Use according to Claim 19, in which the phosphine is trihydroxymethylphosphine and the pH of the stripping composition is about 3.

21. Use according to Claim 19, in which the phosphine is trihydroxypropylphosphine and the pH of the stripping composition is about 9.

22. Process for stripping artificial colour from keratin fibres, **characterized in that** a composition as defined in any one of Claims 1 to 21 is applied to the said keratin fibres for an action time that is sufficient to strip the artificial colour from the keratin fibres.

23. Multi-compartment device for dyeing and then stripping artificial colour from keratin fibres, **characterized in that** it comprises a first compartment containing a composition comprising at least one dye precursor and/or a dye, and a second compartment containing a stripping composition as defined in any one of Claims 1 to 21.

24. Device according to Claim 23, in which the dye precursor(s) is (are) chosen from oxidation bases and/or couplers.

25. Device according to Claim 23 or 24, in which the dye(s) is (are) chosen from direct dyes.

26. Device according to any one of Claims 23 to 25, also comprising a third compartment containing an oxidizing composition.

## Patentansprüche

1. Verwendung einer Zusammensetzung, die in einem kosmetisch akzeptablen Medium mindestens ein Phosphin oder eines seiner Additionssalze mit einer Säure als hauptsächliches Reduktionsmittel enthält, zum Entfernen der künstlichen Farbe aus Keratinfasern.

2. Verwendung nach Anspruch 1, wobei das oder die Phosphin(e) unter den Verbindungen der folgenden Formel (I) ausgewählt sind: 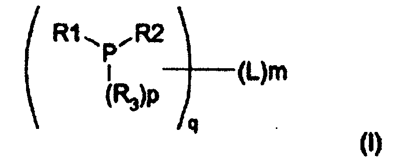 worin bedeuten:
• L eine Verbindungsgruppe, die eine kovalente Bindung oder eine zweiwertige Kohlenwasserstoffgruppe bedeutet, die gegebenenfalls ein oder mehrere Heteroatome enthält, die unter einem Sauerstoffatom, Schwefelatom, Stickstoffatom oder Siliciumatom ausgewählt sind;
• m 0 oder 1;
• q 1 oder 2;
• p 0 oder 1;
• R₁, R₂ und R₃, die gleich oder verschieden sind:
- ein Wasserstoffatom;
- ein Halogenatom;
- eine Hydroxygruppe;
- eine Carboxygruppe;
- eine einwertige Kohlenwasserstoffgruppe, die gegebenenfalls ein oder mehrere Heteroatome enthält, die unter Schwefel, Sauerstoff, Stickstoff, Phosphor oder Silicium ausgewählt sind, und gegebenenfalls mit einer oder mehreren Gruppen substituiert ist, die ausgewählt sind unter:
- einem Halogenatom,
- Hydroxy,
- Alkoxy,
- Halogenalkyl,
- Amino,
- Carboxy,
- Alkoxycarbonyl,
- Amido,
- Alkylaminocarbonyl,
- Acylamino,
- Mono- oder Di(alkyl)amino,
- Mono- oder Di(hydroxyalkyl)amino,
- N-Aryl-N-alkylamino,
- einem aromatischen oder heteroaromatischen Ring, der unsubstituiert vorliegt oder mit einer oder mehreren Gruppen substituiert ist, die unter einem Halogenatom, Hydroxy, Alkoxy, Mono- oder Di(alkyl)amino ausgewählt sind,
- Cyano,
- einer Gruppe, die die Löslichkeit des Phosphins in Wasser erhöht,
- einer aromatischen oder nicht aromatischen, substituierten oder unsubstituierten heterocyclischen Gruppe;
- eine unsubstituierte oder substituierte Arylgruppe;
- eine unsubstituierte oder substituierte Arylalkylgruppe;
- eine Arylalkyloxygruppe;
- eine aromatische oder nicht aromatische, substituierte oder nicht substituierte heterocyclische Gruppe;
- eine Silylgruppe;
mit der Maßgabe, dass:
• wenn q = 1, m = 0 und p = 1;
• wenn q = 2, m = 1 und p = 0 oder 1, mit:
- wenn p = 0, ist die Verbindungsgruppe L an das Phosphoratom gebunden;
- wenn p = 1, ist die Verbindungsgruppe L an eine der Gruppen R₁, R₂ und R₃ gebunden.

3. Verwendung nach Anspruch 2, wobei die Gruppen R₁, R₂ und R₃ nicht gleichzeitig ein Wasserstoffatom bedeuten.

4. Verwendung nach Anspruch 2 oder 3, wobei mindestens eine der Gruppen R₁, R₂ oder R₃ eine Kohlenwasserstoffgruppe bedeutet, die unter den gegebenenfalls substituierten Alkylgruppen ausgewählt ist.

5. Verwendung nach einem der Ansprüche 2 bis 4, wobei die Gruppen R₁, R₂ und R₃ ausgewählt sind unter: einem Wasserstoffatom; einer Alkylgruppe; einer Cycloalkylgruppe, die gegebenenfalls mit einer oder mehreren Alkylgruppen substituiert ist; einer Alkoxygruppe; einer Alkoxyalkylgruppe; einer Halogenalkylgruppe; einer Cyanoalkylgruppe; einer Hydroxyalkylgruppe; einer Carboxyalkylgruppe; einem Halogenatom; einer Hydroxygruppe; einer Carboxygruppe; einer Alkenylgruppe; einer Mono- oder Dialkylaminogruppe; einer N-Aryl-N-alkylaminoalkylgruppe; einer Arylgruppe, die gegebenenfalls mit einer oder mehreren Gruppen substituiert ist, die unter Alkyl, Alkoxy, Mono- oder Dialkylamino, Mono- oder Dialkylaminoalkyl, Halogenalkyl, Hydroxy, Carboxy, einem Halogenatom, einer mit einer Mono- oder Dialkylaminoalkylgruppe substituierten Arylgruppe ausgewählt sind; einer Arylalkylgruppe; einer Arylalkyloxygruppe; einer Pyrrolidinogruppe; einer Furylgruppe; einer Morpholinogruppe; einer Thienylgruppe; einer Pyridylgruppe; einer Trialkylsilylgruppe; einer Alkylgruppe, die mit einer Pyrrolidinogruppe, Furylgruppe, Morpholinogruppe oder Thienylgruppe substituiert ist.

6. Verwendung nach Anspruch 5, wobei die Gruppen R₁, R₂ und R₃, die gleich oder verschieden sind, ausgewählt sind unter: einem Wasserstoffatom; Methyl; Ethyl; Propyl; Isopropyl; *n*-Butyl; Isobutyl; *t*-Butyl; Octyl; Cyclohexyl; Cyclopentyl; Methoxy; Ethoxy; Methoxypropyl; Chlorethyl; Cyanoethyl; Hydroxymethyl; Hydroxypropyl; Carboxyethyl; einem Chloratom; Hydroxy; Carboxy; Trifluormethyl; Chlormethyl; Allyl; Vinyl; Dimethylamino; Diethylamino; Di(isopropyl)amino; Phenyl; *o*-Tolyl; *m*-Tolyl; *p*-Tolyl; Dimethylphenyl; Trimethylphenyl; *o*-Methoxyphenyl; *m*-Methoxyphenyl; *p*-Methoxyphenyl; Dimethoxyphenyl; Trimethoxyphenyl; *o*-(Dimethylamino)phenyl; *m*-(Dimethylamino)-phenyl; *p*-(Dimethylamino)phenyl; Di(*t*-butyl)phenyl; Tri(*t*-butyl)-phenyl; Trifluormethylphenyl; Bis(trifluormethyl)phenyl; o-Fluorphenyl; *m*-Fluorphenyl; *p*-Fluorphenyl; *o*-Chlorphenyl; *m*-Chlorphenyl; *p*-Chlorphenyl; *o*-Hydroxyphenyl; *m*-Hydroxyphenyl; *p*-Hydroxyphenyl; 4-(Diethylaminomethyl)phenyl; 3,5-Dimethyl-4-methoxyphenyl; 2-Methyl-biphenyl; Benzyl; Benzyloxy; Naphthyl; Morpholino; Morpholinomethyl; Pyrrolidino; Furyl; Pyridyl; Thienyl; Trimethylsilyl; 2-(4-Diethylaminomethylphenyl)-phenyl; 5-Methyl-2-isopropylcyclohexyl; N-Methyl-N-phenylaminomethyl; Carboxyphenyl.

7. Verwendung nach einem der vorhergehenden Ansprüche, wobei das Phosphin oder die Phosphine unter den Monophosphinen ausgewählt sind.

8. Verwendung nach einem der Ansprüche 2 bis 7, wobei das oder die Phosphin(e) der Formel (I) so vorliegen, dass q 1 bedeutet.

9. Verwendung nach einem der vorhergehenden Ansprüche, wobei das oder die Phosphin(e) ausgewählt sind unter: Tri(hydroxymethyl)phosphin, Tri(hydroxypropyl)phosphin, Bis(hydroxymethyl)(phenyl)phosphin; Allyldiphenylphosphin; Benzyldiphenylphosphin; Bis(3,4,5-trimethoxyphenyl)chlorphosphin; Bis(3,4,5-trimethoxyphenyl)phosphin; Benzyloxy(diisopropylamino)methylphosphin; Bis(diisopropylamino)chlorphosphin; Bis(2-cyanoethyl)phosphin; Bis(3,5-di-t-butylphenyl)chlorphosphin; Bis(3,5-di-t-butylphenyl)phosphin; Bis(diethylamino)-methylphosphin; Bis(diethylamino)chlorphosphin; Bis(diethylamino)phenylphosphin; Bis(3,5-dimethyl-4-methoxyphenyl)-chlorphosphin; Bis(3,5-dimethyl-4-methoxyphenyl)phosphin; Bis(3,5-dimethylphenyl)chlorphosphin; Bis(3,5-dimethylphenyl)-diethylaminophosphin; Bis(3,5-dimethylphenyl)phosphin; Bis(3,5-ditrifluormethylphenyl)chlorphosphin; Bis(3,5-ditrifluor-methylphenyl)phosphin; Bis(4-fluorphenyl)chlorphosphin; Bis(2-furyl)chlorphosphin; Bis(2-furyl)phosphin; Bis(hydroxymethyl)-phenylphosphin; Bis(4-methoxyphenyl)phenylphosphin; Bis(3,5-dimethylphenyl)phosphin; Bis(3,5-di-t-butylphenyl)chlorphosphin; Bis(3,5-di-t-butylphenyl)phosphin; Bis(3,5-ditrifluormethylphenyl)chlorphosphin; Bis(3,5-ditrifluormethylphenyl)-phosphin; Bis(4-fluorphenyl)chlorphosphin; Bis(4-methoxyphenyl)chlorphosphin; Bis(4-methoxyphenyl)phenylphosphin; Bis(4-methylphenyl)chlorphosphin; Bis(4-methylphenyl)-phosphin; Bis(4-trifluormethylphenyl)chlorphosphin; Bis(4-trifluormethylphenyl)phosphin; Bis(diethylamino)methylphosphin; Bis(diethylamino)phenylphosphin; Bis(hydroxymethyl)phenylphosphin; Bis(o-tolyl)chlorphosphin; Bis(o-tolyl)phosphin; Bis(pyrrolidino)methylphosphin; Butyldichlorphosphin; Butyldiphenylphosphin; t-Butyldiphenylphosphin; Cyclohexyl(diethylamino)chlorphosphin; Cyclohexyl(dimethylamino)chlorphosphin; Cyclohexyldichlorphosphin; Cyclohexyldiphenylphosphin; 2-Chlorethyldiphenylphosphin; 2-(Dicyclohexylphosphino)biphenyl; 2-Dicyclohexylphosphino-2'-(N,N-dimethylamino)biphenyl; Diethylaminodiethylphosphin; Dimethylaminodichlorphosphin; (4-Dimethylaminophenyl)diphenylphosphin; N-[(Diiphenylphosphinyl)methyl]-N-methylanilin; o-Diphenylphosphinobenzoesäure; 2-Methoxy(dichlorphosphino)benzol; 4-Methoxyphenyl(diethylamino)chlorphosphin; 4-Methoxyphenyl(dimethylamino)chlorphosphin; (2-Methoxyphenyl)methylphenylphosphin; 2-Methoxyphosphinobenzol; (5-Methyl-2-isopropylcyclohexyl)diphenylphosphin; Triphenylphosphin; Diallylphenylphosphin; Dibenzylphosphin; Dibutylphenylphosphin; Dibutylphosphin; Dicyclohexylchlorphosphin; Dicyclohexylphenylphosphin; Dicyclohexylphosphin; Diethylchlorphosphin; Diethylphenylphosphin; Diethylphosphin; Diisobutylphosphin; Düsopropylchlorphosphin; Diisopropylphosphin; Dimethyl(phenyl)phosphin; Dimethyl(trimethylsilyl)phosphin; Dimethylchlorphosphin; Diphenyl(*o*-tolyl)-phosphin; Diphenyl(*p*-tolyl)phosphin; Diphenyl(trimethylsilyl)phosphin; Diphenylchlorphosphin; Diphenylphosphin; Diphenylpropylphosphin; Diphenylvinylphosphin; Di-*t*-butylchlorphosphin; Di-*t*-butylhydroxyphosphin; Di-*t*-butylmethylphosphin; Di-*t*-butylphenylphosphin; Di-*t*-butylphosphin; Divinylphenylphosphin; Ethyldichlorphosphin; Ethyldiphenylphosphin; Isopropyldichlorphosphin; Methoxydiethoxyphosphin; Methyldichlorphosphin; Methyldiphenylphosphin; Methylphenylchlorphosphin; Phenylphosphin; Propyldichlorphosphin; *t*-Butyl-bis(trimethylsilyl)phosphin; *t*-Butyldichlorphosphin; *t*-Butyldiethylphosphin; *t*-Butyldiphenylphosphin; *t*-Butylphosphin; Tri(*m*-tolyl)phosphin; Tri(*o*-tolyl)phosphin; Tri(*p*-tolyl)phosphin; Tricyclohexylphosphin; Tricyclopentylphosphin; Triethylphosphin; Triisobutylphosphin; Triisopropylphosphin; Trimethylphosphin; Tri-*n*-butylphosphin; Tri-*n*-octylphosphin; Tripropylphosphin; Tris(1-naphthyl)phosphin; Tris(2,4,6-trimethylphenyl)phosphin; Tris(2,6-dimethoxyphenyl)phosphin; Tris(2-carboxyethyl)phosphin; Tris(2-cyanoethyl)phosphin; Tris(2-furyl)phosphin; Tris(2-methoxyphenyl)phosphin; Tris(2-thienyl)phosphin; Tris(3,5-dimethyl4-methoxy)phosphin; Tris(3-chlorphenyl)phosphin; Tris(3-fluorphenyl)phosphin; Tris(3-methoxyphenyl)phosphin; Tris(3-methoxypropyl)phosphin; Tris(4-chlorphenyl)phosphin; Tris(4-fluorphenyl)phosphin; Tris(4-methoxyphenyl)phosphin; Tris(4-morpholino)phosphin; Tris(hydroxymethyl)phosphin; Tris(trimethylsilyl)phosphin; Tris[3,5-bis(trifluormethyl)phenyl]phosphin; Tri-*t*-butylphosphin; 2-Cyanoethyldiphenylphosphin; 2-Dicyclohexylphosphino-2'-methylbiphenyl; Bis(2,4,6-trimethylphenyl)phosphin; und 2-(Di-t-butylphosphino)biphenyl.

10. Verwendung nach Anspruch 9, wobei das oder die Phosphin(e) unter Trihydroxymethylphosphin; Trihydroxypropylphosphin; und Bis(hydroxymethyl)phenylphosphin ausgewählt sind.

11. Verwendung nach einem der Ansprüche 1 bis 6, wobei das oder die Phosphin(e) unter den Diphosphinen ausgewählt sind.

12. Verwendung nach einem der Ansprüche 2 bis 6 und 11, wobei das oder die Phosphin(e) der Formel (I) so vorliegen, dass q 2 bedeutet.

13. Verwendung nach Anspruch 12, wobei p 0 ist und die Verbindungsgruppe L eine kovalente Bindung bedeutet oder eine zweiwertige Gruppe ist, die unter Binaphthylen; Methylen; Ethylen; Propylen, Butylen; Pentylen; Hexylen; Phenylen; *m*-Dimethylenbenzol; N-Methyl-N'-methylhydrazo; Vinylen; und Diethylenoxy ausgewählt ist.

14. Verwendung nach einem der Ansprüche 1 bis 6 und 11 bis 13, wobei das oder die Phosphin(e) ausgewählt sind unter: 2,2'-Bis(dicyclohexylphosphino)-1, 1'-binaphthyl; 2,2'-Bis[bis(3,5-dimethylphenylphosphino)]-1,1'-binaphthyl; 1,4-Bis[bis(3,5-dimethylphenyl)phosphino]butan; 1,2-Bis[bis(3,5-dimethylphenyl)phosphino]ethan; Bis[bis(3,5-dimethylphenyl)phosphino]-methan; 1,5-Bis[bis(3,5-dimethylphenyl)phosphino]pentan; 1,3-Bis[bis(3,5-dimethylphenyl)phosphino]propan; 2,2'-Bis[bis(3,5-ditrifluormethylphenyl)phosphino]-1,1'-binaphthyl; 1,4-Bis[bis-(3,5-ditrifluormethylphenyl)phosphino]butan; 1,2-Bis[bis(3,5-ditrifluormethylphenyl)phosphino]ethan; Bis[bis(3,5-ditrifluormethylphenyl)phosphino]methan; 1,5-Bis[bis(3,5-ditrifluormethylphenyl)phosphino]pentan; 1,3-Bis[bis(3,5-ditrifluormethylphenyl)phosphino]propan; 1,2-Bis(di-*t*-butylphosphino)-benzol; 1,4-Bis(di-*t*-butylphosphino)butan; 1,2-Bis(di-*t*-butylphosphino)ethan; 1,3-Bis(di-*t*-butylphosphinomethyl)benzol; 1,3-Bis(di-*t*-butylphosphino)propan; 1,2-Bis(dichlorphosphino)-benzol; 1,3-Bis(dichlorphosphino)-benzol; 1,4-Bis(dichlorphosphino)benzol; 1,4-Bis(dichlorphosphino)butan; 1,2-Bis-(dichlorphosphino)-1,2-dimethylhydrazin, 1,2-Bis(dichlorphosphino)ethan; Bis(dichlorphosphino)methan; 1,3-Bis(dichlorphosphino)propan; 1,2-Bis(dicycohexylphosphino)benzol, 2,2'-Bis(dicyclohexylphosphino)-1,1'-binaphthyl; 1,4-Bis(dicyclohexylphosphino)butan; (2R,3R)-Bis(dicyclohexylphosphino)-butan; (2S,3S)-Bis(dicyclohexylphosphino)butan; 1,2-Bis(dicyclohexylphosphino)ethan; Bis(dicyclohexylphosphino)methan; 1,3-Bis(dicyclohexylphosphino)propan; Bis[2-(4-diethylaminomethylphenyl)-phenylphosphino]ethylether; 1,2-Bis(diethylphosphino)ethan; 1,2-Bis(dimethylphosphino)benzol; 1,4-Bis(dimethylphosphino)butan; 1,2-Bis(dimethylphosphino)ethan; Bis(dimethylphosphino)methan; 1,3-Bis(dimethylphosphino)-propan; 1,2-Bis(diphenylphosphino)benzol; 1,3-Bis(diphenylphosphino)benzol; 1,4-Bis(diphenylphosphino)benzol; 2,2'-Bis(diphenylphosphino)-1,1'-binaphthyl; 1,4-Bis(diphenylphosphino)butan; 1,2-Bis(diphenylphosphino)ethan; cis-1,2-Bis(diphenylphosphino)ethylen; trans-1,2-Bis(diphenylphosphino)ethylen; Bis(2-diphenylphosphino)ethylether; 1,6-Bis(diphenylphosphino)hexan; Bis(diphenylphosphino)methan; 1,5-Bis(diphenylphosphino)pentan; 1,3-Bis(diphenylphosphino)-propan; 1,2-Bis(ditrifluormethylphosphino)ethan; 1,2-Bis[(2-methoxyphenyl)phenylphosphino]ethan; 1,2-Bis(phenylphosphino)ethan; 1,3-Bis(phenylphosphino)propan; Bis-2-[(phenyl)(3-pyridyl)phosphinoethyl]ether; 1,2-Bis(phosphino)-benzol; 1,2-Bis(phosphino)ethan; Bis(phosphino)methan; 1,2-Bis(trifluormethyl)phosphino)ethan; Bis(di-*t*-butylphosphino)-pentan; Tetraphenylbiphosphin.

15. Verwendung nach einem der vorhergehenden Ansprüche, wobei das oder die Phosphin(e) Verbindungen sind, die in dem kosmetisch akzeptablen Medium löslich sind.

16. Verwendung nach Anspruch 15, wobei das oder die Phosphin(e) wasserlöslich sind.

17. Verwendung nach einem der vorhergehenden Ansprüche, wobei die Zusammensetzung als einziges Reduktionsmittel das oder die Phosphin(e) enthält.

18. Verwendung nach einem der vorhergehenden Ansprüche, wobei das oder die Phosphin(e) in einer Menge von etwa 0,001 bis 30 Gew.-% des Gesamtgewichts der Zusammensetzung enthalten sind.

19. Verwendung nach einem der vorhergehenden Ansprüche, wobei der pH-Wert der Zusammensetzung zum Abziehen der Farbe im Bereich von 2 bis 12 liegt.

20. Verwendung nach Anspruch 19, wobei es sich bei dem Phosphin um das Trihydroxymethylphosphin handelt und der pH-Wert der Zusammensetzung zum Abziehen der Farbe etwa 3 beträgt.

21. Verwendung nach Anspruch 19, wobei es sich bei dem Phosphin um das Trihydroxypropylphosphin handelt und der pH-Wert der Zusammensetzung zum Abziehen der Farbe etwa 9 beträgt.

22. Verfahren zum Entfernen der künstlichen Farbe aus Keratinfasern, **dadurch gekennzeichnet, dass** auf die Keratinfasern eine Zusammensetzung nach einem der Ansprüche 1 bis 21 während einer Zeitspanne aufgebracht wird, die ausreichend ist, um die künstliche Farbe der Keratinfasern zu entfernen.

23. Vorrichtung mit mehreren Abteilungen, die zum Färben und anschließendem Entfernen der künstlichen Farbe der Keratinfasern vorgesehen ist, **dadurch gekennzeichnet, dass** sie eine erste Abteilung mit einer Zusammensetzung, die mindestens ein Farbstoffvorprodukt eines Farbstoffes und/oder einen Farbstoff enthält, und eine zweite Abteilung mit einer Zusammensetzung zum Abziehen der Farbe nach einem der Ansprüche 1 bis 21 aufweist

24. Vorrichtung nach Anspruch 23, wobei das Farbstoffvorprodukt eines Oxidationsfarbstoffes oder die Farbstoffvorprodukte von Oxidationsfarbstoffen unter den Oxidationsbasen und/oder Kupplern ausgewählt sind.

25. Vorrichtung nach Anspruch 23 oder 24, wobei der Farbstoff oder die Farbstoffe unter den Direktfarbstoffen ausgewählt sind.

26. Vorrichtung nach einem der Ansprüche 23 bis 25, die ferner eine dritte Abteilung mit einer oxidierenden Zusammensetzung aufweist.
